(19) Europäisches Patentamt
European Patent Office
Office européen des brevets

(11) EP 0 534 817 B1

(12) **FASCICULE DE BREVET EUROPEEN**

(45) Date de publication et mention de la délivrance du brevet: **09.12.1998 Bulletin 1998/50**

(51) Int. Cl.$^6$: **C07C 67/11**, C07C 69/88, C07D 213/79

(21) Numéro de dépôt: **92402484.7**

(22) Date de dépôt: **11.09.1992**

(54) **Procédé d'esterification de sels d'acides carboxyliques**

Verfahren zur Veresterung von Salzen von Carbonsäuren

Esterification process of salts of carboxylic acids

(84) Etats contractants désignés:
**DE ES FR GB IE**

(30) Priorité: **24.09.1991 FR 9111747**

(43) Date de publication de la demande:
**31.03.1993 Bulletin 1993/13**

(60) Demande divisionnaire:
**98104242.7**

(73) Titulaire: **RHODIA CHIMIE**
**92408 Courbevoie Cédex (FR)**

(72) Inventeur: **Crochemore, Michel**
**F-69630 Chaponost (FR)**

(74) Mandataire:
**Dutruc-Rosset, Marie-Claude et al**
**RHODIA SERVICES**
**Direction de la Propriété Industrielle**
**25, quai Paul Doumer**
**92408 Courbevoie Cédex (FR)**

(56) Documents cités:
**FR-A- 2 331 545**
**FR-A- 2 430 401**
**GB-A- 916 772**

Remarques:
Le dossier contient des informations techniques présentées postérieurement au dépôt de la demande et ne figurant pas dans le présent fascicule.

EP 0 534 817 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet un procédé d'estérification de sels d'acides carboxyliques. Plus précisément, l'invention concerne l'esterification de sels d'acides aussi bien monocarboxyliques que polycarboxyliques aromatiques.

Il a été proposé, selon US-A-3 341 575, de préparer des esters à partir d'halogénures d'alkyle et de sels d'acides carboxyliques, dans un solvant de type amide, notamment, le diméthylformamide, en présence d'un composé iodé.

Il a également été préconisé, selon FR-A-1 294 105, d'effectuer la préparation d'esters alkyliques d'acides carboxyliques, par réaction d'un acide carboxylique avec des quantités équimoléculaires d'un halogénure d'alkyle et d'une amine aliphatique tertiaire. Selon ce procédé, les esters dérivés des acides ou anhydrides suivants ont été préparés : acides phtaliques, caproïque, adipique, anhydride succinique, anhydride maleïque, anhydride phtalique, phtalate acide de n-butyle, acide éthyl-4 mercapto-alpha-hydroxybutyrique...

Les différents procédés décrits font intervenir des solvants et l'on aboutit à des procédés polluants, peu productifs.

Il est décrit dans GB-A 916 772, un procédé de préparation d'un ester d'acide dicarboxylique par réaction d'une solution aqueuse d'un sel d'un acide dicarboxylique et d'un halogénure d'alkyle difficilement hydrolysable, en présence d'un sel d'ammonium quaternaire comme catalyseur.

Il est aussi connu de préparer selon FR-A 2 430 401, l'acétate de m-phénoxybenzyle, par mélange du chlorure de m-phénoxybenzyle, de l'acétate de sodium, d'un catalyseur de transfert de phase et d'eau.

Par ailleurs, l'estérification des acides carboxyliques présentant un encombrement stérique an alpha ou au voisinage de la fonction carboxyle présente des difficultés et souvent, des vitesses réactionelles lentes sont observées.

Un des objectifs de la présente invention est de fournir un procédé d'estérification de sels d'acides carboxyliques aromatiques qui permet d'éviter les inconvénients précités.

Un autre objectif de l'invention est de disposer d'un procédé qui s'applique à une large gamme de sels d'acides carboxyliques, aromatiques notamment à ceux qui sont difficiles à estérifier.

La présente invention a pour objet un procédé d'estérification d'un sel d'un acide carboxylique aromatique caractérisé par le fait qu'il consiste à conduire la réaction, en l'absence de tout solvant organique, entre un sel d'un acide carboxylique aromatique et un dérivé halogéné d'un hydrocarbure aliphatique, cycloaliphatique ou aliphatique porteur d'un substituant cyclique, en présence d'un catalyseur de transfert de phase et en présence d'eau : le sel de l'acide carboxylique aromatique étant introduit progressivement dans le mélange réactionnel comprenant le dérivé halogéné et le catalyseur, la réaction ayant lieu à une température d'au plus 120°C, de préférence comprise entre 50°C et 120°C, conduisant à un milieu comprenant une phase aqueuse et une phase organique comprenant l'ester de l'acide carboxylique aromatique.

Conformément au procédé de l'invention, on fait réagir le sel d'acide carboxylique aromatique et le dérivé halogéné en milieu aqueux et en l'absence de tout solvant organique.

Il a été trouvé, de manière inattendue, qu'il était possible, selon le procédé de l'invention, de préparer des esters d'acides carboxyliques, aromatiques notamment d'acides benzoïques en milieu aqueux, avec un excellent rendement. Ce résultat est tout à fait surprenant car on aurait pu s'attendre à une hydrolyse du dérivé halogéné gui aurait entraîné une baisse du rendement de la réaction.

On utilisera ci-après le terme générique de "dérivé halogéné" pour désigner les composés halogénés précités.

Par l'expression "catalyseur de transfert de phase", on entend un catalyseur capable de faire passer l'anion carboxylate de la phase aqueuse à la phase organique.

Le procédé de l'invention s'applique à tout sel dérivé d'un acide carboxylique mono- ou polycarboxylique aromatiques tels que ; les acides carbocycliques ou hétérocycliques, monocycliques ou polycycliques.

Il est très bien adapté à la préparation d'esters d'acides benzoïques en particulier ceux présentant un encombrement au voisinage de la fonction carboxyle.

Dans l'exposé gui suit de la présente invention, on entend par composé aromatique, la notion classique d'aromaticité telle que définie dans la littérature, notamment par Jerry MARCH - Advanced Organic Chemistry, 3ème édition, John Wiley and Sons, 1985 p. 37 et suivantes.

On entend par acide benzoïque, tout composé aromatique portant au moins une fonction COOH.

D'une manière générale, le sel d'acide carboxylique peut être symbolisé par la formule (I) suivante :

$$[Q - COO^{-}]_{w} M^{w+} \qquad (I)$$

dans ladite formule (I) :

- Q représente un radical hydrocarboné, aromatique éventuellement substitué comportant de 1 à 40 atomes de carbone,
- M représente un cation métallique.
- w représente la valence du métal M.

Les sels d'acides carboxyliques aromatiques mis en oeuvre préférentiellement répondent à la formule (I) dans laquelle Q représente un radical hydrocarboné, éventuellement substitué comportant de 1 à 20 atomes de carbone et M représente un cation métallique du groupe Ia ou IIa de la classification périodique ou un radical ammonium.

N'importe quel sel d' acide carboxylique aromatique peut être mis en oeuvre dans la réaction de l'invention dans la mesure où il présente une solubilité dans l'eau suffisante pour permettre de conduire aisement le procédé de l'invention.

D'un point de vue pratique et économique, on fait appel aux sels métalliques du groupe (Ia), à savoir aux métaux alcalins et, de préférence, au sodium et au potassium ; aux sels métalliques du groupe (IIa), c'est-à-dire aux métaux alcalino-terreux et plus particulièrement, au magnésium, au calcium ou au baryum ; ou aux sels d'ammonium.

Dans la formule (I), w est égal de préférence à 1 ou 2.

Selon l'invention, on met en oeuvre comme matière première de départ, un sel d'acide carboxylique aromatique répondant à la formule (I) dans laquelle le reste Q représente un radical hydrocarboné aromatique, substitué ou non qui peut être un radical carbocyclique ou hétérocyclique, monocyclique ou polycyclique.

Conviennent tout particulièrement bien à la mise en oeuvre du procédé de l'invention, les sels des acides carboxyliques de formule générale (I) dans laquelle Q représente un reste hydrocarboné aromatique monocyclique ou polycyclique.

Q représente un reste hydrocarboné aromatique, et notamment benzénique répondant à la formule générale (II) :

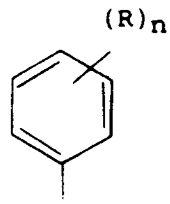

(II)

dans ladite formule (II) :

- n est un nombre entier de 0 à 5, de préférence de 0 à 3,
- R représente $R_1$, l'un des groupes ou fonctions suivantes :

  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un radical de formule

$$—R_2—OH$$

$$-R_2-COOM$$

$$-R_2-CHO$$

$$-R_2-NO_2$$

$$-R_2-CN$$

$$-R_2-NH_2$$

$$-R_2-SH$$

$$-R_2-X$$

$$-R_2-CF_3$$

dans lesdites formules, $R_2$ représente un lien valentiel ou un radical hydrocarboné divalent, linéaire ou ramifié, saturé ou insaturé, ayant de 1 à 4 atomes de carbone tel que, par exemple, méthylène, éthylène, propylène, isopropylène, isopropylidène ; X symbolise un atome d'halogène, de préférence un atome de chlore, de brome ou de fluor et M ayant la signification donnée précédemment.

- R représente $R_3$ l'un des radicaux plus complexes suivants :

  . un radical

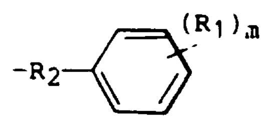

  dans lequel $R_1$ et $R_2$ ont la signification donnée précédemment et m est un nombre entier de 0 à 3,

  . un radical $—R_2—A—R_4$ dans lequel $R_2$ a la signification donnée précédemment, $R_4$ représente un radical alkyle linéaire ou ramifié ayant de 1 à 6 atomes de carbone, de préférence, de 1 à 4 atomes de carbone ou un radical

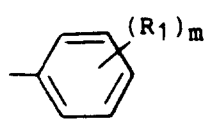

  et A symbolise l'un des groupes suivants :
  —O—, —CO—,

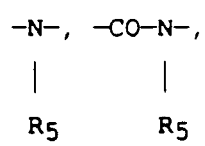

  —COO—, —S—, $SO_2$—, dans ces formules, $R_5$ représente un atome d'hydrogène ou un radical alkyle linéaire ou ramifié ayant de 1 à 4 atomes de carbone, de préférence, un radical méthyle ou éthyle.

Lorsque n est supérieur à 1, les radicaux R peuvent être identiques ou différents et 2 atomes de carbone successifs du cycle benzénique peuvent être reliés entre eux par un pont cétalique tels que les radicaux méthylène dioxy ou éthylène dioxy extranucléaires.

De préférence, n est égal à (0)—1—2 ou 3.

Parmi tous les restes Q précités, on met en oeuvre tout préférentiellement dans le procédé de l'invention, les sels d'acides carboxyliques répondant à la formule genérale (I) dans laquelle Q représente un reste aromatique répondant à la formule générale (II) dans laquelle :

- n est égal à 0, 1, 2 ou 3,
- R représente l'un des groupes ou fonctions suivantes :

  . un radical alkyle linéaire ou ramifié, ayant de 1 à 4 atomes de carbone,
  . un radical alkoxy linéaire ou ramifié ayant de 1 à 4 atomes de carbone,
  . un radical méthylène ou éthylène dioxy,
  . un groupe —OH,
  . un groupe —CHO,
  . un radical phényle ou benzyle,
  . un atome d'halogène.

Encore plus préférentiellement, on choisit les composés de formule (I) dans laquelle les radicaux R identiques ou différents sont un atome d'hydrogène, un groupe hydroxyle, un radical méthyle, un radical méthoxy, un groupe —CHO.

Q peut également représenter un reste hydrocarboné aromatique polycyclique ; les cycles pouvant former entre eux des systèmes ortho- condensés, ortho- et péri-condensés. On peut citer plus particulièrement, un reste naphtalénique ; lesdits cycles pouvant être substitués par 1 à 4 radicaux $R_1$, de préférence 1 à 3, $R_1$ ayant les significations énoncées précédemment pour les substituants du reste hydrocarboné aromatique de formule générale (II).

Dans la formule générale (I) des sels d'acides carboxyliques, Q peut également représenter un reste hétérocyclique, aromatique, comportant notamment 5 ou 6 atomes dans le cycle dont 1 ou 2 hétéroatomes tels que les atomes d,azote, de soufre et d'oxygène ; les atomes de carbone de l'hétérocycle pouvant éventuellement être substitués, dans leur totalité ou pour une partie d'entre eux seulement par des radicaux $R_1$, $R_1$ ayant les significations énoncées précédemment pour les substituants du reste hydrocarboné aromatique de formule générale (II).

Q peut aussi représenter un reste hétérocyclique aromatique polycyclique défini comme étant soit un radical constitué par au moins 2 hétérocycles aromatiques ou non contenant au moins un hétéroatome dans chaque cycle et formant entre eux des systèmes ortho- ou ortho- et péricondensés ou soit un radical constitué par au moins un cycle hydrocarboné aromatique ou non et au moins un hétérocycle aromatique ou non formant entre eux des systèmes ortho- ou ortho- et péricondensés ; l'un des deux étant aromatique les atomes de carbone desdits cycles pouvant éventuellement être substitués, dans leur totalité ou pour une partie d'entre eux seulement par des radicaux $R_1$, $R_1$ ayant les significations énoncées précédemment pour les substituants du reste hydrocarboné aromatique de formule générale (II).

A titre de sels d'acides carboxyliques aromatiques comprenant au moins un groupe carboxylique répondant à la formule (I), on fait appel plus particulièrement aux sels dérivant des acides carboxyliques suivants :

- les acides carboxyliques hétérocycliques aromatiques tels que les acides furannecarboxyliques, thiophènecarboxyliques, pyrrolecarboxyliques, pyrazinecarboxyliques, l'acide nicotinique, isonicotinique, l'acide picolinique, l'acide pipéronylique,
- les acides carboxyliques carbocycliques aromatiques tels que l'acide benzoïque, phtalique, isophtalique, téréphtalique, les acides naphtalènecarboxyliques, les acides toluiques,
- les acides hydroxybenzoïques suivants : l'acide hydroxy-2 benzoïque (acide salicylique), l'acide hydroxy-3 benzoïque, l'acide hydroxy-4 benzoïque, l'acide méthyl-3 salicylique, l' acide méthyl-4 salicylique, l'acide méthyl-5 salicylique, l'acide hydroxy-3 méthyl-4 benzoïque, l'acide méthoxy-3 salicylique, l'acide méthoxy-4 salicylique, l'acide méthoxy-5 salicylique, l'acide hydroxy-3 méthoxy-4 benzoïque (acide isovanillique), l'acide hydroxy-4 méthoxy-3 benzoïque (acide vanillique), l'acide hydroxy-3 diméthoxy-4,5 benzoïque, l'acide hydroxy-4 diméthoxy-3,5 benzoïque (acide syringique), l'acide hydroxy-5 isophtalique, l'acide amino-3 salicylique, l'acide amino-4 salicylique, l'acide amino-5 salicylique, l'acide hydroxy-3 amino-2 benzoïque, l'acide nitro-3 salicylique, l'acide hydroxy-3 nitro-4 benzoïque, l'acide hydroxy-4 nitro-3 benzoïque, l'acide hydroxy-3 méthyl-4 nitro-2 benzoïque, l'acide diiodo-3,5 salicylique, l'acide dihydroxy-2,3 benzoïque, l'acide dihydroxy-2,4 benzoïque, l'acide dihydroxy-2,5 benzoïque, l'acide dihydroxy-2,6 benzoïque, l'acide dinydroxy-3,4 benzoïque (acide protocatéchique), l'acide dihydroxy-3,5 benzoïque, l'acide dihydroxy-3,5 méthyl-4 benzoïque, l'acide trihydroxy-2,3,4 benzoïque, l'acide trihydroxy-2,4,6 benzoïque, lac de trihydroxy-3,4,5 benzoïque,
- les alcoxy- et phénoxyacides tels que l'acide phénoxy-4 benzoïque, l'acide (carboxy-4 phénoxy-4) benzoïque,
- les oxo-acides tels que l'acide acétyl-2 benzoïque, l'acide acétyl-4 benzoïque, l'acide benzoyl-2 benzoïque, l'acide benzoyl-4 benzoïque,
- les acyloxy-acides tels que l'acide acétoxy-2 benzoïque, l'acide acétoxy-4 benzoïque,
- les amido-acides tels que l'acide acétamido-2 benzoïque, l'acide acétamido-3 benzoïque, l'acide N-acétamido-4 benzoïque,

Parmi tous les composés cités précédemment à titre illustratif et sans caractère limitatif, le procédé de l'invention s'applique particulièrement bien aux composés suivants :

- l'acide salicylique et l'acide hydroxy-4 benzoïque.
- l'acide benzoïque et ses dérivés substitués par un groupe alkyle $C_1$-$C_4$, acétoxy, acétamido,
- l'acide nicotinique.

Le procédé de l'invention consiste donc à mettre en contact au moins un des acides carboxyliques précités, sous forme salifiée, avec au moins un dérivé halogéné que l'on peut représenter symboliquement par la formule générale (III) :

$$R_7\text{-}X \qquad \text{(III)}$$

dans ladite formule (III) :

- X est un atome d'halogène : ledit atome d'halogène ne devant pas être en position vinylique ou alcynyle
- $R_7$, éventuellement substitué représente un radical aliphatique acyclique, saturé ou insaturé, linéaire ou ramifié ; un radical cycloaliphatique, saturé ou insaturé, monocylique ou polycyclique ; un radical aliphatique saturé ou insaturé, linéaire ou ramifié, porteur d'un substituant cyclique.

L'atome d'halogène peut être le chlore, le brome ou l'iode mais on choisit, de préférence, les deux premiers.
Le radical $R_7$ représente plus particulièrement :

1° - un radical alkyle, alcényle, alcadiényle, alcynyle, linéaire ou ramifié ayant, de préférence, de 1 à 12 atomes de carbone ; la chaîne hydrocarbonée pouvant être éventuellement :

- interrompue par l'un des groupes suivants :
  -O-, -CO-, -COO-,

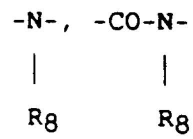

  dans ces formules, $R_8$ représente un atome d'hydrogène ou un radical alkyle ayant de 1 à 4 atomes de carbone, un radical cyclohexyle ou phényle,
- et/ou porteuse de l'un des substituants Y suivants :
  un groupe -OH, un groupe -COOH, un groupe -CHO, un groupe $-NO_2$, un groupe -C≡N, un groupe amine non quaternisable ou N-protégé, un atome d'halogène de préférence, chlore ou brome, un groupe $-CF_3$.

A titre d'exemples de radicaux aliphatiques $R_7$, on peut citer les radicaux méthyle, éthyle, propyle, isopropyle, butyle, isobutyle, sec-butyle, tert-butyle, éthyl-2 butyle, pentyle, isopentyle, néopentyle, tert-pentyle, méthyl-2 pentyle, hexyle, éthyl-2 hexyle ; allyle, méthyl-3 butène-2 yle, hexène-3 yle ; méthoxyméthyle, acétamidométhyle.
2° - un radical cycloalkyle ou cycloalcényle ayant de préférence de 5 à 7 atomes de carbone éventuellement porteur d'un ou plusieurs substituants Y tels que précités et/ou d'un ou plusieurs substituants Z qui peuvent être : un radical alkyle linéaire ou ramifié ayant de 1 à 4 atomes de carbone, un radical alkoxy linéaire ou ramifié ayant de 1 à 4 atomes de carbone, un radical de formule :

$$R_8\text{-CO-},\ R_8\text{-COO-},\ R_8\text{-O-CO-},$$

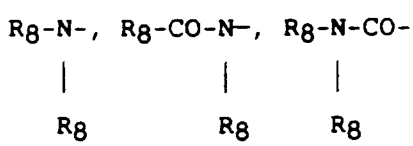

dans lesdites formules, $R_8$ ayant la signification donnée précédemment ; lesdits radicaux pouvant être polycycliques de type "pontés".

Comme exemples de radicaux cycloaliphatiques $R_7$, on peut mentionner le radical cyclohexyle et le radical cyclohexène-1 yle-1.
3° - un radical aliphatique acyclique, saturé ou insaturé, linéaire ou ramifié, tel que précisé sous 1°, porteur d'un substituant cyclique. Par cycle, on entend un cycle carbocyclique ou hétérocyclique, saturé, insaturé ou aromatique.

Le radical aliphatique acyclique peut être relié au cycle par un lien valentiel ou par l'un des groupes suivants :
-O-, -CO-, -COO-,

-N-, -CO-N-
|     |
$R_8$   $R_8$

dans lesdites formules, $R_8$ ayant la signification donnée précédemment.

Comme exemples de cycles, on peut envisager :

a - un radical cycloaliphatique saturé ou insaturé, monocyclique ou polycyclique tel que mentionné sous 2°.
b - un radical phényle, tolyle, xylyle, éventuellement porteur d'un ou plusieurs substituants Y ou Z.
c - un radical hétérocyclique monocyclique, saturé, insaturé ou aromatique et comportant en tant qu'hétéroatomes un ou plusieurs atomes d'oxygène, d'azote ou de soufre, contenant 4 à 6 atomes dans le cycle : ledit radical pouvant être porteur d'un ou plusieurs substituants Y ou Z.

A titre illustratif de radicaux hétérocycliques, on peut citer les radicaux pyrrolidinyle, imidazolidinyl, pipéridyle, furyle, pyrrolyle, thiényle, isoxazolyle, furazannyle, isothiazolyle, imidazolyle, pyrazolyle, pyridyle, pyridazinyle, pyrimidinyle.

d - un radical constitué par un enchaînement de 2 à 4 groupes tels que définis aux paragraphes a et/ou b et/ou c, liés entre eux par un lien valentiel et/ou par l'un des groupes suivants :
-O-, -S-,

-N-, -CO-N-,
|     |
$R_8$   $R_8$

-CO-, -COO-, $-SO_2-$, et/ou encore au moins un groupe de type alcoylène ou alcoylidène ayant de 1 à 4 atomes de carbone ; dans lesdites formules, $R_8$ représente un atome d'hydrogène, un radical alkyle ayant de 1 à 4 atomes de carbone, un radical cyclohexyle ou phényle.

On peut citer plus particulièrement les radicaux biphényle, méthylène-1,1' biphényle, oxy-1,1' biphényle, (carboxy-4 phénoxy-4) phényle, méthylène dioxy-3,4 phényle, acétyl-2 phényle, acétyl-4 phényle, benzoyl-2 phényle, benzoyl-4 phényle, benzoyloxy-3 éthyl-2 phényle, acétoxy-2 phényle, acétoxy-4 phényle, acétamido-2 phényle, acétamido-4 phényle.

e - un radical aromatique constitué par un ensemble de 2 à 3 cycles aromatiques carbocycliques et/ou hétérocycliques et formant entre eux des systèmes orthocondensés.

Les cycles aromatiques sont de préférence des noyaux benzéniques et pyridiniques.

On donne ci-après, à titre illustratif des exemples des radicaux aromatiques polycycliques : les radicaux anthryle, quinolyle, naphtyridinyle, benzofurannyle, indolyle.

Comme exemples de radicaux cyclo- ou arylaliphatiques $R_7$ porteurs d'un substituant cyclique, on peut citer préférentiellement les radicaux cyclohexylméthyle, cyclohexylbutyle, benzyle, phényl-2 éthyle, [(butyl-2)-4 phényl]-2 éthyle, styryle, α-phénylcyclohexylméthyle, phénoxyméthyle, phénoxyéthyle.

Dans la définition du radical $R_7$ du dérivé halogéné de formule (III), il est noté que celui-ci peut porter de un à plusieurs substituants Y ou Z. Par "plusieurs", on entend généralement un nombre total ne dépassant pas 3.

D'une manière préférentielle, le radical $R_7$ représente :

- un radical alkyle linéaire ou ramifié ayant de 1 à 8 atomes de carbone, tel que par exemple, le radical méthyle, éthyle, propyle, isopropyle, butyle, isobutyle, sec-butyle, tert-butyle, éthyl-2 butyle, pentyle, isopentyle, néopentyle, tert-pentyle, méthyl-2 pentyle, hexyle, éthyl-2 hexyle,
- un radical alcényle linéaire ou ramifié ayant de 2 à 8 atomes de carbone et comportant une double liaison éthylénique en β du groupe X comme par exemple, le radical allyle, méthyl-3 butène-2 yle, hexène-3 yle,
- un radical cyclohexyle éventuellement substitué par un radical alcoyle linéaire ou ramifié ayant de 1 à 4 atomes de carbone,
- un radical arylalkyle tel que symbolisé par la formule suivante

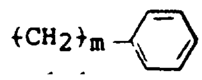

dans laquelle m est un nombre entier allant de 1 à 4, de préférence, égal à 1.

Parmi les dérivés halogénés répondant a' la formule (III) on peut citer tout particulièrement, les composés suivants :

- le bromure de méthyle
- le bromure d'éthyle
- le bromure de n-propyle
- le bromoisopropane
- le bromobutane
- le bromohexane
- le bromoheptane
- le bromooctane
- le bromo-1 méthyl-2 butane
- le bromo-1 méthyl-3 butane
- le bromo-2 méthyl-2 butane
- le bromure d'allyle
- le chlorure d'allyle
- le chlorure de crotyle
- le chloro-3 méthyl-2 propène
- le chloro-1 butène-2
- le chloro-1 méthyl-3 butène-2
- le bromure de prényle
- le bromure de géranyle
- le chlorure de géranyle
- l'$\alpha$-bromoacétone
- l'$\alpha,\alpha$'-dibromoacétone
- l'$\alpha$-chloroacétone
- l'$\alpha,\alpha$'-chloroacétone
- l'$\alpha$-bromopropionate de méthyle
- l'$\alpha$-bromopropionate de butyle
- l'$\alpha$-chloropropionate de méthyle
- l'$\alpha$-chloropropionate de butyle
- le bromocyclobutane
- le bromocyclopentane
- le bromocyclohexane
- le bromocycloheptane
- le (bromométhyl)cyclopropane
- le (chlorométhyl-2)pyridine
- le (chlorométhyl-3)pyridine
- le (chlorométhyl-4)pyridine
- le bromure de benzyle
- le chlorure de benzyle
- le chlorométhyl-4 stilbène
- le (bromométhyl-1) naphtalène
- le (bromométhyl-2) naphtalène

En ce qui concerne le choix de l'halogénure utilisé, soit chlorure ou bromure on le détermine en fonction de la nature de l'hydrocarbure dont dérive l'halogénure et d'impératifs économiques.

On peut faire appel dans la mise en oeuvre du procédé de l'invention, a un composé bromé dans tous les cas.

Toutefois, les chlorures étant les moins chers, on cherchera à les utiliser préférentiellement, mais ceux-ci ne peuvent pas toujours être utilisés. En effet, il n'est pas possible selon l'invention d'avoir recours à un halogénure de type chlorure s'il y a présence d'une insaturation sur la chaîne hydrocarbonée portant l'atome de chlore, en position $\alpha$ par

rapport à ce dernier : l' insaturation pouvant être une double ou une triple insaturation. Par exemple, le chlorure de benzyle peut être avantageusement utilisé.

Il a été mentionné dans la définition du radical $R_7$ que celui-ci pouvait porter un autre atome d'halogène.

Il devient ainsi possible selon le procédé de l'invention d'obtenir des diesters d'acides carboxyliques.

Il y a lieu de remarquer que les atomes d'halogène ne doivent pas être présents sur le même atome de carbone.

Des diesters d'un acide carboxylique peuvent être obtenus dans le cas de la mise en oeuvre :

- de dérivés dibromés quels qu'ils soient, tels que par exemple, le dibromométhane, le dibromo-1,2 éthane, le dibromo-1,2 propane, le dibromo-1,3 propane, le dibromo-1,2 butane, le dibromo-1,3 butane, le dibromo-1,4 butane, le dibromo-1,6 hexane, le dibromo-1,4 butène-2, le dibromo-1,4 butyne, le bis-(bromométhyl)-1,4 benzène,
- de dérivés dichlorés notamment le dichlorométhane, le dichloroéthane, Le dichloro-1,4 pentène-2 le bis-(chlorométhyl)-1,4 benzène,
- de dérivés mixtes bromé et chloré tels que le bromochloroéthane, le bromo-1 chloro-3 propane, le bromo-1 chloro-4 butane.

Selon le procédé de l'invention, il est également possible d'obtenir un diester lorsque le radical Q de l'acide carboxylique, sous forme salifiée, de formule (I) présente un autre groupe -COOM.

Il est à noter que le procédé de l'invention s'applique également pour effectuer l'estérification d'acides polycarboxyliques, quelle que soit la position du groupe carboxylate, qu'il soit porté par un cycle et/ou qu'il soit présent sur une chaîne hydrocarbonée aliphatique.

Intervient donc dans le procédé de l'invention, un catalyseur qui est un catalyseur de transfert de phase.

Dans le procédé de l'invention, on peut faire appel aux catalyseurs de transfert de phase connus, notamment, ceux décrits dans l'ouvrage de Jerry MARCH - Advanced Organic Chemistry, 3ème édition, John Wiley and Sons, 1985 p. 320 et suivantes.

Les catalyseurs préférentiellement mis en oeuvre dans le procédé de l'invention sont les sels d'onium et, plus particulièrement les sels d'ammonium quaternaire et/ou de phosphonium.

Les sels d'oniums susceptibles d'être utilisés dans le procédé de l'invention sont ceux dont les ions onium dérivent notamment de l'azote, du phosphore, de l'arsenic, du soufre, du sélénium, de l'oxygène, du carbone ou de l'iode et coordiné à des restes hydrocarbonés. Les ions onium dérivant de l'azote, du phosphore ou de l'arsenic seront quadricoordinés, les ions onium dérivant du soufre, du selénium, de l'oxygène, du carbone ou de S=O seront tricoordinés tandis que les ions onium dérivant de l'iode seront dicoordinés.

Les restes hydrocarbonés coordinés à ces différents éléments sont des radicaux alkyles, alcényles, aryles, cycloalkyles, aralkyles éventuellement substitués, 2 restes hydrocarbonés coordinés pouvant former ensemble un radical unique divalent.

La nature des anions liés à ces cations organiques n'a pas d'importance critique. Toutes les bases "dures" ou "intermédiaires" conviennent comme anion.

Par base "dure" ou "intermédiaire", on entend tout anion répondant à la définition classique donnée par R. PEARSON dans Journal of Chem. Ed. 45, pages 581 - 587 (1968), les termes "dure" et "intermédiaire" ayant respectivement la signification des termes de "hard" et "borderline" utilisés dans cette référence.

Parmi les ions onium pouvant être utilisés dans le présent procédé de l'invention, conviennent particulièrement ceux répondant à l'une des formules générales suivantes :

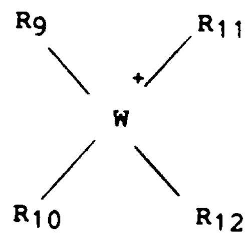

(IV)

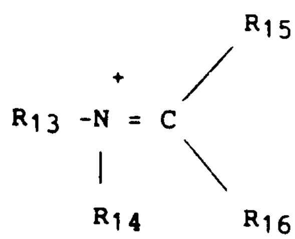

(V)

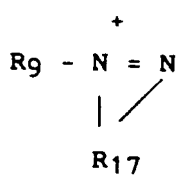

(V bis)

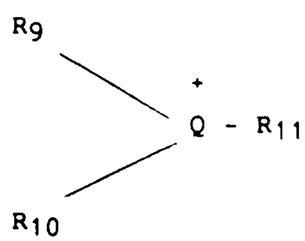

(VI)

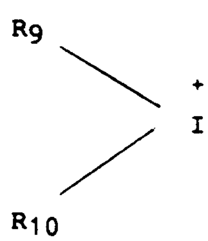

(VII)

dans lesdites formules :

- W représente N, P ou As ;
- Q représente S, O, Se, S=O ou C ;
- $R_9$, $R_{10}$, $R_{11}$ et $R_{12}$, identiques ou différents représentent :

  . un radical alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;

. un radical alcényle, linéaire ou ramifié, ayant 2 à 12 atomes de carbone ;
. un radical aryle ayant 6 à 10 atomes de carbone, éventuellement substitué par un ou plusieurs groupements ou atomes alkyle ayant 1 à 4 atomes de carbone, alkoxy, alkoxycarbonyle, le radical alkoxy ayant 1 à 4 atomes de carbone, ou halogène,
. deux desdits radicaux $R_9$ à $R_{12}$ pouvant former ensemble un radical alkylène, alcénylène ou alcadiènylène, linéaire ou ramifié ayant de 3 à 6 atomes de carbone ;

- $R_{13}$, $R_{14}$, $R_{15}$, $R_{16}$ sont identiques ou différents et représentent :

  . un radical alkyle linéaire ou ramifié contenant de 1 à 4 atomes de carbone ;
  . les radicaux $R_{15}$ et $R_{16}$ pouvant former ensemble un radical alkylène contenant de 3 à 6 atomes de carbone ;
  . les radicaux $R_{14}$ et $R_{15}$ ou $R_{14}$ et $R_{16}$ pouvant former ensemble un radical alkylène, alcénylène ou alcadiènylène, contenant 4 atomes de carbone et constituant avec l'atome d'azote un hétérocycle azoté ;

- $R_{17}$ représente un radical divalent formant avec les 2 atomes d'azote un cycle ayant 4 à 6 atomes pouvant comporter un ou plusieurs atomes d'azote, de soufre et/ou d'oxygène, ledit cycle pouvant être substitué par un ou plusieurs radicaux tels que $R_9$.

Parmi les bases "dures" ou "intermédiaires" pouvant constituer l'anion desdits sels d'onium, on peut citer les ions : $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, $Ph\text{-}SO_3^-$, $HSO_4^-$, $NO_3^-$, $SO_4^{2-}$, $Cl^-$, $Br^-$, $I^-$, $OH^-$, Ph représentant un radical phényle, ainsi que tous les autres anions répondant à la définition de base "dure" ou "intermédiaire" de PEARSON.

Pour des raisons de commodité de mise en oeuvre, lesdits anions pourront être choisis parmi $PO_4^{3-}$, $HPO_4^{2-}$, $H_2PO_4^-$, $CH_3SO_3^-$, $Ph\text{-}SO_3^-$, $NO_3^-$, $SO_4^{2-}$, $PF_6^-$, $Cl^-$, $Br^-$, $I^-$, Ph ayant la signification précédente. On recourra avantageusement aux anions $Br^-$ et $Cl^-$.

A titre d'exemples d'ions onium répondant à la formule (IV), on peut citer les cations :

- tétraméthylammonium,
- triéthylméthylammonium,
- tributylméthylammonium,
- triméthylpropylammonium,
- tétraéthylammonium,
- tétrabutylammonium,
- dodécyltriméthylammonium,
- méthyltrioctylammonium,
- heptyltributylammonium,
- tétrapropylammonium,
- tétrapentylammonium,
- tétrahexylammonium,
- tétraheptylammonium,
- tétraoctylammonium,
- tétradécylammonium,
- butyltripropylammonium,
- méthyltributylammonium,
- pentyltributylammonium,
- méthyldiéthylpropylammonium,
- éthyldiméthylpropylammonium,
- tétradodécylammonium,
- tétraoctadécylammonium,
- hexadécyltriméthylammonium,
- benzyltriméthylammonium,
- benzyldiméthylpropylammonium,
- benzyldiméthyloctylammonium,
- benzyltributylammonium,
- benzyltriéthylammonium,
- phényltriméthylammonium,
- benzyldiméthyltétradécylammonium,

- benzyldiméthylhexadécylammonium,
- diméthyldiphénylammonium,
- méthyltriphénylammonium,
- butène-2-yltriéthylammonium,
- N,N-diméthyl-tétraméthylèneammonium,
- N,N-diéthyl-tétraméthylèneammonium,
- tétraméthylphosphonium,
- tétrabutylphosphonium,
- éthyltriméthylphosphonium,
- triméthylpentylphosphonium,
- octyltriméthylphosphonium,
- dodécyltriméthylphosphonium,
- triméthylphénylphosphonium,
- diéthyldiméthylphosphonium,
- dicyclohexyldiméthylphosphonium,
- diméthyldiphénylphosphonium,
- cyclohexyltriméthylphosphonium,
- triéthylméthylphosphonium,
- méthyltri(isopropyl)phosphonium,
- méthyltri(n-propyl)phosphonium,
- méthyltri(n-butyl)phosphonium,
- méthyltri(méthyl-2 propyl)phosphonium,
- méthyltricyclohexylphosphonium,
- méthyltriphénylphosphonium,
- méthyltribenzylphosphonium,
- méthyltri(méthyl-4 phényl) phosphonium,
- méthyltrixylylphosphonium,
- diéthylméthylphénylphosphonium,
- dibenzylméthylphénylphosphonium,
- éthyltriphénylphosphonium,
- tétraéthylphosphonium,
- éthyltri(n-propyl)phosphonium,
- triéthylpentylphosphonium,
- hexadécyltributylphosphonium,
- éthyltriphénylphosphonium,
- n-butyltri(n-propyl)phosphonium,
- butyltriphénylphosphonium,
- benzyltriphénylphosphonium,
- (β-phényléthyl)diméthylphénylphosphonium,
- tétraphénylphosphonium,
- triphényl(méthyl-4 phényl)phosphonium,
- tétrakis(hydroxyméthyl)phosphonium,
- tétraphénylarsonium.

Parmi les cations répondant aux formules (V) et (V bis) on peut citer les cations :

- N-méthylpyridinium,
- N-éthylpyridinium,
- N-hexadécylpyridinium,
- N-méthylpicolinium,
- triphényl-1,2,4 triazolium.

A titre d'exemples d'ions onium répondant à la formule (VI), on peut citer les cations :

- triméthylsulfonium,
- triéthylsulfonium,
- triphénylsulfonium,
- triméthylsulfoxonium,

- triphénylcarbénium,
- triéthyloxonium.

A titre d'exemples d'ions onium répondant à la formule (VII), on peut citer les cations :

- diphényliodonium,
- diméthoxy-4,4' diphényliodonium (ou les composés décrits dans JACS 1958, 81, 342),
- diphényliodonium 2-carboxylate

Parmi les ions onium oui peuvent être utilisés dans le cadre du présent procédé, on préférera le plus souvent les ions ammonium quaternaire, les ions phosphonium quaternaire, les ions sulfonium et les ions iodonium.

Conviennent tout particulièrement bien, les ions ammonium dont les quatre groupes sont des radicaux alkyle ayant de 1 à 5 atomes de carbone ou un radical benzyle.

En ce qui concerne le choix de l'anion, on préfèrera $Br^-$ ou $Cl^-$.

Les catalyseurs convenant tout à fait bien à la présente invention sont le chlorure ou le bromure de tributylbenzylammonium ou phosphonium, le chlorure ou le bromure de tétraméthylammonium ou phosphonium, le chlorure ou le bromure de tétraéthylammonium ou phosphonium, le chlorure ou le bromure de tétrabutylammonium ou phosphonium.

Le sel d' onium peut être introduit au cours du procédé de l'invention, à l'état solide ou sous forme d'une solution dans l'un de ses solvants, le plus souvent l'eau.

Conformément au procédé de l'invention, la réaction d'estérification du sel de l'acide carboxylique aromatique est effectuée en présence d'un catalyseur de transfert de phase ; les différents réactifs étant mis en oeuvre généralement dans les proportions définies ci-après.

Le rapport molaire entre le nombre de fonctions carboxylate de l'acide carboxylique aromatique et le nombre d'atomes d'halogène réagissants du dérivé halogéné varie de préférence entre 0,5 et 2,0. Il est choisi préférentiellement aux environs de 1,0. Lorsque l'on s'écarte de cette valeur préférentielle et que l'on met en oeuvre un défaut de dérivé halogéné, la réaction est moins complète. Dans le cas ou le derivé halogéné est en excès, il y a lieu de séparer ledit excès en fin de réaction.

En ce qui concerne la quantité de catalyseur utilisée, celle-ci varie avantageusement de telle sorte que le rapport molaire entre ledit catalyseur et le sel de l'acide carboxylique varie entre 0,01 et 0,50, de préférence, entre 0,05 et 0,2. La borne supérieure n'est pas critique et peut être largement dépassée sans inconvénient car le catalyseur peut être éventuellement recyclé en fin de réaction.

Comme mentionné précédemment, la réaction est conduite, en milieu aqueux, en l'absence de tout solvant organique.

Le sel de l'acide carboxylique aromatique est mis en solution dans l'eau. Sa concentration n'est pas critique. Elle dépend uniquement de sa solubilité dans l'eau.

Selon un mode préférentiel de réalisation de l'invention, on choisit une concentration aussi élevée que possible. On peut citer, à titre d'exemples, que la concentration du sel de l'acide carboxylique dans l'eau peut atteindre 50 % en poids. Il est souhaitaole que la concentration se situe entre 30 et 50 % en poids Toutefois, des concentrations très inférieures peuvent être utilisées.

La quantité d'eau présente dans le milieu réactionnel représente généralement de 50 à 100 % du poids total des réactifs engagés.

Dans son mode de réalisation préférentielle, l'invention a pour objet un procédé d'estérification d'un sel d'un acide carboxylique aromatique caractérisé par le fait qu'il consiste à faire réagir un sel d'un acide carboxylique aromatique avec un dérivé halogéné d'un hydrocarbure aliphatique, cycloaliphatique ou aliphatique porteur d'un substituant cyclique, en milieu aqueux, en présence d'un catalyseur de transfert de phase ; le sel de l'acide carboxylique, aromatique en solution aqueuse, étant introduit progressivement dans le dérivé halogéné.

Selon un mode préféré de réalisation pratique de l'invention, on prépare un pied de cuve comprenant le dérivé halogéné et le catalyseur de la réaction.

On effectue ensuite l'addition dans ledit milieu, du sel de l'acide carboxylique en solution aqueuse.

L'addition du sel de l'acide carboxylique aromatique est faite de manière progressive. Sa durée d' addition peut varier largement entre 5 minutes et 8 heures, de préférence, entre 20 minutes et 4 heures.

La température à laquelle est mise en oeuvre le procédé de l'invention est d'au plus 120°C, de préférence entre 50°C et 120°C.

D'une manière préférentielle, la température est choisie entre 70°C et 90°C.

La pression réactionnelle n'est pas critique et est généralement la pression atmosphérique.

Pour atteindre les températures indiquées précédemment, on travaille de préférence sous pression autogène.

La durée de la réaction dépend de la température réactionnelle et du taux de transformation désiré. Lorsque la température est choisie dans la zone préférentielle, la durée de la réaction peut varier dans de larges limites, par exemple, de 15 minutes à 10 heures.

En fin de réaction, l'ester de l'acide carboxylique aromatique constitue la phase organique qui peut être séparée de la phase aqueuse.

La phase organique peut contenir également le catalyseur et le dérivé halogéné qui n'a pas réagi dans le cas où il est mis en oeuvre en excès.

La phase aqueuse recueillie contient un sel sous forme d'halogénure dérivant du cation métallique provenant de l'acide carboxylique et de l'anion qui est l'halogénure provenant du dérivé halogéné.

On peut isoler l'ester obtenu de la phase organique, selon les techniques classiquement utilisées telles que, par exemple, la distillation ou l'extraction à l'aide d'un solvant adéquat.

Le procédé de l'invention convient tout à fait bien à la préparation des esters de l'acide salicylique, notamment : les salicylates d'alkyle ou d'alcényle tels que par exemple, le salicylate de méthyle, le salicylate d'éthyle, le salicylate d'isopropyle, le salicylate d'amyle ou d'isoamyle, le salicylate de méthyl-2 pentyle, le salicylate de n-hexyle, le salicylate d'éthyl-2 butyle, le salicylate d'éthyl-2 hexyle, le salicylate de méthyl-2 butène-2 yle, le salicylate de cis-hexène-3 yle ; les salicylates d'alkyle ou d'alcényle porteur d'un substituant comme par exemple, le salicylate de glycol, le salicylate de monométhylamine, le salicylate de β-isopropoxyéthyle ; les salicylates de cycloalkyle, de cycloalcényle, éventuellement substitués tels que par exemple, le salicylate de cyclohexyle, le salicylate d'isopropyl-2 cyclohexyle, le salicylate de triméthyl-3,3,5 cyclohexyle, le salicylate de bornyle ; les salicylates d'arylalkyle comme, par exemple, le salicylate de benzyle.

L'invention s'applique également bien à la préparation des esters de l'acide o- m- ou p-aminosalicylique.

On peut mentionner notamment l'o-amino et monosalicylate de menthyle, le p-amino et monosalicylate de menthyle, le p-amino et monosalicylate d'allyle.

Le procédé de l'invention est également bien adapté à la préparation des esters de l'acide p-hydroxybenzoique. On peut citer plus particulièrement le p-hydroxybenzoate de méthyle, le p-hydroxybenzoate de propyle.

L'un des intérêts du procédé, objet de la présente invention est qu'il permet d'obtenir les esters des acides hydroxybenzoïques avec un bon taux de transformation de l'acide de départ et d'obtenir une bonne sélectivité par rapport à la réaction de O-alkylation possible.

Le procédé de l'invention convient également tout à fait bien à la préparation des esters d'alkyle, de cyclohexyle et de benzyle de l'acide benzoïque et de ses dérivés substitués par un groupe alkyle $C_1$-$C_4$, acétoxy, acétamido ; de l'acide nicotinique.

Les exemples qui suivent illustrent l'invention sans toutefois la limiter.

Dans les exemples, les abréviations suivantes signifient :

$$TT_H = \frac{\text{nombre de moles de dérivé halogéné transformées}}{\text{nombre de moles de dérivé halogéné introduites}} \%$$

$$TT_A = \frac{\text{nombre de moles de sel de l'acide carboxylique transformées}}{\text{nombre de moles de sel de l'acide carboxylique introduites}} \%$$

$$RR_H = \frac{\text{nombre de moles d'ester de l'acide carboxylique formées}}{\text{nombre de moles de dérivé halogéné introduites}} \%$$

$$RR_A = \frac{\text{nombre de moles d'ester de l'acide carboxylique formées}}{\text{nombre de moles de sel de l'acide carboxylique introduites}} \%$$

$$RT_H = \frac{\text{nombre de moles d'ester de l'acide carboxylique formées}}{\text{nombre de moles de dérivé halogéné transformées}} \%$$

$$RT_A = \frac{\text{nombre de moles d'ester de l'acide carboxylique formées}}{\text{nombre de moles de sel de l'acide carboxylique transformées}} \%$$

Les exemples 1 à 7 illustrent la préparation de différents esters d'acides carboxyliques.

Exemple 1

Préparation de salicylate d'allyle

Dans un réacteur tel que précédemment décrit, on charge 60,5 g de bromure d'allyle, 10 ml d'eau et 15,6 g de chlorure de tributylbenzyl ammonium (0,05 mol) et l'on chauffe à 60°C.

On introduit progressivement, à 60°C, en 6 heures une solution de salicylate de sodium préparée à partir de 69 g d'acide salicylique (0,5 mol), 25 ml d'eau et 50 ml d'une solution aqueuse de soude 10 N.

On maintient ensuite le milieu réactionnel, à 60°C encore 20 minutes.

En fin de réaction, on sépare le salicylate d'allyle selon le mode opératoire de l'exemple précédent.

On confirme la présence de salicylate d'allyle par RMN et spectrographie de masse.

Les résultats obtenus sont les suivants :

- taux de transformation ($TT_H$) du bromure d'allyle = 100 %
- taux de transformation ($TT_A$) du salicylate de sodium = 100 %
- rendement en salicylate d'allyle par rapport au bromure d'allyle =
  . $RR_H$ = 90 %
- rendement en salicylate d'allyle par rapport au salicylate de sodium =
  . $RR_A$ = 90 %

Exemple 2

Préparation de salicylate de benzyle

Dans un réacteur en verre de 500 ml muni d'un système d'agitation, d'un dispositif de contrôle de température, d'un dispositif d'introduction de réactif, d'un réfrigérant et équipé d'un système de chauffage, on charge 63,3 g (0,5 mol) de chlorure de benzyle, le catalyseur qui est le bromure de tétrabutylammonium à raison de 10 % en moles par rapport au salicylate de sodium et l'on chauffe à 80°C.

On ajoute 20 ml d'eau et l'on introduit progressivement, à 80°C, 80 g (0,5 mol) de salicylate de sodium en solution dans 60 ml d'eau.

On coule ladite solution dans le chlorure de benzyle contenant le catalyseur, en 2 heures 35 minutes.

On maintient ensuite le milieu réactionnel, à 80°C pendant 3 heure 30 minutes.

En fin de réaction et après refroidissement, on sépare la phase organique (64 ml) qui contient le salicylate de benzyle et le catalyseur de la phase aqueuse (58 ml) qui contient du chlorure de sodium.

A partir de cette couche organique et après lavage à l'eau par 3 fois 50 ml d'eau, on récupère le salicylate de benzyle par distillation à 148°C sous 2,5 mm de mercure.

Les résultats obtenus sont les suivants :

- taux de transformation ($TT_A$) du salicylate de sodium = 98,6 %
- taux de transformation ($TT_H$) du chlorure de benzyle = 99,8 %
- rendement du salicylate de benzyle par rapport au salicylate de sodium =
  . $RR_A$ = 92,5 %
  . $RT_A$ = 93,8 %
- rendement en salicylate de benzyle par rapport au chlorure de benzyle =
  . $RR_H$ = 92,7 %
  . $RT_H$ = 93,9 %

Exemple comparatif 3

Exemples 4 et 5

Préparation de salicylate de benzyle

On reproduit l'exemple 2, en l'absence de catalyseur (exemple comparatif 3).
On conduit d'autres essais dans lesquels on met en oeuvre un catalyseur d'une autre nature qui sont :

- le chlorure de tributylbenzylammonium $BzBu_3NCl$ (exemple 4)
- le bromure de tétraéthylammonium $Et_4NBr$ (exemple 5)

Les quantités de réactifs mises en jeu, les conditions opératoires et les résultats obtenus sont consignés dans le tableau I suivant :

Tableau I

| Réf. ex. | SNa | BzCl | Rapport mol. SNa/BzCl | eau | catalyseur | coulée SNa | température °C | durée réaction | rendements/SNa | | | rendements/BzCl | | |
|---|---|---|---|---|---|---|---|---|---|---|---|---|---|---|
| | | | | | | | | | $TT_A$ | $RR_A$ | $RT_A$ | $TT_H$ | $RR_H$ | $RT_H$ |
| 3 | 80 g 0,5 mol | 63,3 g 0,5 mol | 1 | 120 ml | sans | 0 h 30 | 80°C | 3 h 30 | 62,0 % | 34,2 % | 55,1 % | 97,4 % | 34,2 % | 35,1 % |
| 4 | 80 g 0,5 mol | 63,3 g 0,5 mol | 1 | 120 ml | $BzBu_3NCl$ 10 % | 5 h 45 | 80°C | 3 h 30 | 96,6 % | 93,4 % | 97,0 % | 99,8 % | 93,4 % | 93,6 % |
| 5 | 80 g 0,5 mol | 63,3 g 0,5 mol | 1 | 120 ml | $Et_4NBr$ 10 % | 0 h 20 | 80°C | 3 h 30 | 65,2 % | 47,3 % | 72,5 % | 98,5 % | 47,3 % | 48,0 % |

Dans le tableau I, les abréviations signifient :
- SNa : salicylate de sodium
- BzCl : chlorure de benzyle

On note les très mauvais rendements obtenus en l'absence de catalyseur.

Exemple comparatif 6

Préparation de salicylate de benzyle

Dans un réacteur en verre tel que décrit dans l'exemple 1, on charge 80 g (0,5 mol) de salicylate de sodium en solution dans 60 ml d'eau, le catalyseur qui est le bromure de tétrabutylammonium à raison de 10 % en moles par rapport au salicylate de sodium et l'on chauffe à 70°C.

On ajoute 20 ml d'eau et l'on introduit progressivement, à 80°C, 63,3 g (0,5 mol) de chlorure de benzyle.

On coule le chlorure de benzyle dans la solution de salicylate de sodium contenant le catalyseur en 2 heures 30 minutes.

On maintient ensuite le milieu réactionnel 1 heure à 70°C et 2 heures à 80°C.

En fin de réaction et après refroidissement, on sépare la phase organique qui contient le salicylate de benzyle et le catalyseur de la phase aqueuse qui contient du chlorure de sodium.

On traite la phase organique comme dans l'exemple 1.

Les résultats obtenus sont les suivants :

- taux de transformation ($TT_A$) du salicylate de sodium = 86,8 %
- taux de transformation ($TT_H$) du chlorure de benzyle = 100 %
- rendement du salicylate de benzyle par rapport au salicylate de sodium =
  . $RR_A$ = 76,7 %
  . $RT_A$ = 88,4 %
- rendement en salicylate de benzyle par rapport au chlorure de benzyle =
  . $RR_H$ = 76,7 %
  . $RT_H$ = 76,7 %

Exemple 7

Préparation de nicotinate de benzyle

Dans un réacteur tel que précédemment décrit, on charge 25,3 g de chlorure de benzyle, 10 ml d'eau et 6,24 g de chlorure de tributylbenzylammonium et l'on chauffe à 80°C.

On introduit progressivement à 80°C, en 45 minutes, une solution de nicotinate de sodium préparée à partir de 24,6 g d'acide nicotinique, 75 ml d'eau et 20 ml d'une solution aqueuse de soude 10 N.

On maintient ensuite le milieu réactionnel, à 100°C encore 3 heures.

En fin de réaction, on sépare le nicotinate de benzyle selon le même mode opératoire que l'exemple précédent.

On confirme la présence de nicotinate de benzyle par RMN et spectrographie de masse.

Les résultats obtenus sont les suivants :

- taux de transformation ($TT_H$) du chlorure de benzyle = 100 %
- taux de transformation ($TT_A$) du nicotinate de sodium = 100 %
- rendement en nicotinate de benzyle par rapport au chlorure de benzyle =
  . $RR_H$ = 100 %
- rendement en nicotinate de benzyle par rapport au nicotinate de sodium =
  . $RR_A$ = 100 %

**Revendications**

1. Procédé d'estérification d'un sel d'un acide carboxylique aromatique caractérisé pu le fait qu'il consiste à conduire la réaction, en l'absence de tout solvant organique, entre un sel d'un acide carboxylique aromatique et un dérivé halogéné d'un hydrocarbure aliphatique, cycloaliphatique ou aliphatique porteur d'un substituant cyclique, en présence d'un catalyseur de transfert de phase et en présence d'eau : le sel de l'acide carboxylique aromatique étant introduit progressivement dans le mélange réactionnel comprenant le dérivé halogéné et le catalyseur, la réaction

ayant lieu à une température d'au plus 120°C, de préférence comprise entre 50°C et 120°C, conduisant à un milieu comprenant une phase aqueuse et une phase organique comprenant l'ester de l'acide carboxylique.

**2.** Procédé selon la revendication 1 caractérisé par le fait que le sel de l'acide carboxylique répond à la formule (I) suivante :

$$[Q - COO^-]_w M^{w+} \qquad (I)$$

dans ladite formule (I) :

- Q représente un radical hydrocarboné aromatique, éventuellement substitué comportant de 1 à 40 atomes de carbone,
- M représente un cation métallique,
- w représente la valence du métal M.

**3.** Procédé selon l'une des revendications 1 et 2 caractérisé par le fait que le sel de l'acide carboxylique répond à la formule (I) dans laquelle Q représente un radical hydrocarboné, aromatique eventuellement substitué comportant de 1 à 20 atomes de carbone et M représente un cation métallique du groupe Ia ou IIa de la classification périodique ou un radical ammonium.

**4.** Procédé selon l'une des revendications 1 à 3 caractérisé par le fait que le sel de l'acide carboxylique répond à la formule (I) dans laquelle le reste Q représente un radical hydrocarboné, substitué ou non qui est un radical carbocyclique ou hétérocyclique, monocyclique ou polycyclique.

**5.** Procédé selon l'une des revendications 1 à 4 caractérisé par le fait que le sel de l'acide carboxylique répond à la formule (I) dans laquelle Q représente un reste hydrocarboné aromatique répondant à la formule générale (II) :

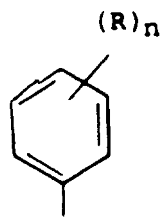

(II)

dans ladite formule (II) :

- n est un nombre entier de 0 à 5, de préférence de 0 à 3,
- R représente $R_1$, l'un des groupes ou fonctions suivantes :
  - . un radical alkyle, linéaire ou ramifié, ayant de 1 à 6 atomes de carbone, de préférence de 1 à 4 atomes de carbone,
  - . un radical alcényle linéaire ou ramifié ayant de 2 à 6 atomes de carbone, de préférence, de 2 à 4 atomes de carbone,
  - . un radical alkoxy linéaire ou ramifié ayant de 1 à 6 atomes de carbone, de préférence de 1 à 4 atomes de carbone,
  - . un radical de formule

$$-R_2-OH$$

$$-R_2-COOM$$

$$-R_2-CHO$$

$$-R_2-NO_2$$

$$-R_2-CN$$

$-R_2-NH_2$

$-R_2-SH$

$-R_2-X$

$-R_2-CF_3$

dans lesdites formules $R_2$ représente un lien valentiel ou un radical hydrocarboné divalent, linéaire ou ramifié, saturé ou insaturé, ayant de 1 à 4 atomes de carbone ; X symbolise un atome d'halogène, de préférence un atome de chlore, de brome ou de fluor et M ayant la signification donnée précédemment,

- R représente $R_3$ l'un des radicaux plus complexes suivants :

  . un radical

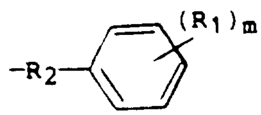

dans lequel $R_1$ et $R_2$ ont la signification donnée précédemment et m est un nombre entier de 0 à 3,

  . un radical $—R_2—A—R_4$ dans lequel $R_2$ a la signification donnée précédemment, $R_4$ représente un radical alkyle linéaire ou ramifiée ayant de 1 à 6 atomes de carbone, de préférence, de 1 à 4 atomes de carbone ou un radical

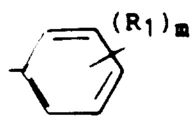

et A symbolise l'un des groupes suivants :
—O—, —CO—, —COO—,

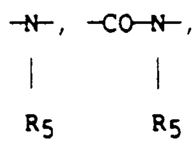

—S—, $—SO_2—$, dans ces formules $R_5$ représente un atome d'hydrogène ou un radical alkyle linéaire ou ramifié ayant de 1 à 4 atomes de carbone, de préférence un radical méthyle ou éthyle.

**6.** Procédé selon la revendication 5 caractérisé par le fait que le sel de l' acide carboxylique répond à la formule (I) dans laquelle Q représente un reste hydrocarboné aromatique répondant à la formule générale (II) dans laquelle n est supérieur à 1, les radicaux R sont identiques ou différents et 2 atomes de carbone successifs du cycle benzénique peuvent être reliés entre eux par un pont cétalique, de préférence, un radical méthylène dioxy ou éthylène dioxy extranucléaire.

**7.** Procédé selon la revendication 5 caractérisé par le fait que le sel de l'acide carboxylique répond à la formule générale (I) dans laquelle Q représente un reste hydrocarboné aromatique répondant à la formule générale (II) dans laquelle :

- n est égal à 0, 1, 2 ou 3,
- R représente l'un des groupes ou fonctions suivantes :

  . un radical alkyle linéaire ou ramifié, ayant de 1 à 4 atomes de carbone,

. un radical alkoxy linéaire ou ramifié ayant de 1 à 4 atomes de carbone,
. un radical méthylène ou éthylène dioxy,
. un groupe —OH,
. un groupe —CHO,
. un radical phényle ou benzyle,
. un atome d'halogène.

**8.** Procédé selon l'une des revendications 1 à 4 caractérisé par le fait que le sel de l'acide carboxylique répond à la formule générale (I) dans laquelle Q représente un reste naphtalénique ; lesdits cycles pouvant être substitués par 1 à 4 radicaux $R_1$, de préférence 1 à 3, $R_1$ ayant les significations énoncées précédemment dans la revendication 5.

**9.** Procédé selon l'une des revendications 1 à 4 caractérisé par le fait que le sel de l'acide carboxylique répond à la formule (I) dans laquelle Q représente un reste hétérocyclique, aromatique, conportant notamment 5 ou 6 atomes dans le cycle dont 1 ou 2 hétéroatomes tels que les atomes d'azote, de soufre et d'oxygène ou un reste hétérocyclique polycyclique aromatique ; les atomes de carbone desdits cycles pouvant éventuellement être substitués, dans leur totalité ou pour une partie d'entre eux seulement par des radicaux $R_1$, $R_1$ ayant la signfication donnée précédemment dans la revendication 5.

**10.** Procédé selon l'une des revendications 1 à 9 caractérisé par le fart que le sel de l'acide carboxylique répondant à la formule (I) est choisi parmi les acides carboxyliques suivants :

- les acides carboxyliques hétérocycliques aromatiques,
- les acides carboxyliques carbocycliques aromatiques,
- les hydroxy-acides aromatiques,
- les alcoxy-et phénoxyacides aromatiques,
- les oxo-acides aromatiques,
- les acyloxy-acides aromatiques,
- les amido-acides aromatiques.

**11.** Procédé selon l'une des revendications 1 à 4 caractérisé par le fait que le sel de l'acide carboxylique répond à la formule générale (I) est choisi parmi les acides carboxyliques hétérocycliques aromatiques tels que les acides furannecarboxyliques, thiophènecarboxyliques, pyrrolecarboxyliques, pyrazinecarboxyliques, lac de nicotinque, isonicotinique, l'acide picolinique, lac de pipéronylique ; les acides carboxyliques carbocycliques aromatiques tels que l'acide benzoïque, phtalique, isophtalique, téréphtalique, les acides naphtalènecarboxyliques, les acides toluiques.

**12.** Procédé selon l'une des revendications 1 à 4 caractérisé par le fait que le sel de l'acide carboxylique répondant à la formule générale (I) est choisi parmi les acides hydroxybenzoïques suivants : l'acide hydroxy-2 benzoïque (acide salicylique), l'acide hydroxy-3 benzoïque, l'acide hydroxy-4 benzoïque, l'acide méthyl-3 salicylique, l' acide méthyl-4 salicylique, l'acide méthyl-5 salicylique, l'acide hydroxy-3 méthyl-4 benzoïque, l'acide méthoxy-3 salicylique, l'acide méthoxy-4 salicylique, l'acide méthoxy-5 salicylique, l'acide hydroxy-3 méthoxy-4 benzoïque (acide isovanillique), l'acide hydroxy-4 méthoxy-3 benzoïque (acide vanillique), l'acide hydroxy-3 diméthoxy-4,5 benzoïque, l'acide hydroxy-4 diméthoxy-3,5 benzoïque (acide syringique), l'acide hydroxy-5 isophtalique, l'acide amino-3 salicylique, l'acide amino-4 salicylique, l'acide amino-5 salicylique, l'acide hydroxy-3 amino-2 benzoïque, l'acide nitro-3 salicylique, l'acide hydroxy-3 nitro-4 benzoïque, lac de hydroxy-4 nitro-3 benzoïque, l'acide hydroxy-3 méthyl-4 nitro-2 benzoïque, l'acide diiod-3,5 salicylique, l'acide dihydroxy-2,3 benzoïque, l'acide dihydroxy-2,4 benzoïque, l'acide dihydroxy-2,5 benzoïque, l'acide dihydroxy-2,6 benzoïque, l'acide dihydroxy-3,4 benzoïque (acide protocatéchique), l'acide dihydroxy-3,5 benzoïque, l'acide dihydroxy-3,5 méthyl-4 benzoïque, l'acide trihydroxy-2,3,4 benzoïque, l'acide trihydroxy-2,4,6 benzoïque, l'acide trihydroxy-3,4,5 benzoïque.

**13.** Procédé selon l'une des revendications 1 à 4 caractérisé par le fait que le sel de l'acide carboxylique répondant à la formule (I) est choisi parmi les acides benzoïques et dérivés tels que l'acide phénoxy-4 benzoïque, l'acide (carboxy-4 phénoxy-4) benzoïque, l'acide acétyl-2 benzoïque, lac de acétyl-4 benzoïque, l'acide benzoyl-2 benzoïque, l'acide benzoyl-4 benzoïque, l'acide acétoxy-2 benzoïque, l'acide acétoxy-4 benzoïque, l'acide acétamido-2 benzoïque, l'acide acétamido-3 benzoïque, l'acide N-acétamido-4 benzoïque.

**14.** Procédé selon l'une des revendications 1 à 13 caractérisé par le fait que le sel de l'acide carboxylique répondant à

la formule (I) est choisi parmi les acides carboxyliques suivants :

- l'acide salicylique et l'acide hydroxy-4 benzoïque.
- l'acide benzoïque et ses dérivés substitués par un groupe alkyle $C_1$-$C_4$, acétoxy, acétamido,
- l'acide nicotinique.

**15.** Procédé selon l'une des revendications 1 à 14 caractérisé par le fait que le dérivé halogéné répond à la formule (III) suivante :

$$R_7\text{-}X \qquad \text{(III)}$$

dans ladite formule (III) :

- X est un atome d'halogène : ledit atome d'halogène ne devant pas être en position vinylique ou alcynyle
- $R_7$, éventuellement substitué, représente un radical aliphatique acyclique, saturé ou insaturé, l\néaire ou ramifié ; un radical cycloaliphatique, saturé ou insaturé, monocylique ou polycyclique ; un radical aliphatique saturé ou insature, linéaire ou ramifié porteur d'un substituant cyclique.

**16.** Procédé selon la revendication 15 caractérisé par le fait que le dérivé halogéné répond à la formule (III) dans laquelle le radical $R_7$ représente :

1° - un radical alkyle, alcényle, alcadiényle, alcynyle, linéaire ou ramifié ayant, de préférence, de 1 à 12 atomes de carbone ; la chaîne hydrocarbonée pouvant être éventuellement :

- interrompue par l'un des groupes suivants :
  -O-, -CO-, -COO-,

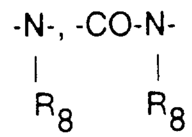

  dans ces formules, $R_8$ représente un atome d'hydrogène ou un radical alkyle ayant de 1 à 4 atomes de carbone, un radical cyclohexyle ou phényle,
- et/ou porteuse de l'un des substituants Y suivants :
  un groupe -OH, un groupe -COOH, un groupe -CHO, un groupe $-NO_2$, un groupe -C≡N, un groupe amine non quaternisable ou N-protégé, un atome d'halogène de préférence, chlore ou brome, un groupe $-CF_3$.

2° - un radical cycloalkyle ou cycloalcényle ayant de préférence de 5 à 7 atomes de carbone eventuellement porteur d'un ou plusieurs substituants Y tels que précités et/ou d'un ou plusieurs substituants Z qui peuvent être : un radical alkyle linéaire ou ramifié ayant de 1 à 4 atomes de carbone, un radical alkoxy linéaire ou ramifié ayant de 1 à 4 atomes de carbone, un radical de formule :

$$R_8\text{-CO-},\ R_8\text{-COO-},\ R_8\text{-O-CO-},$$

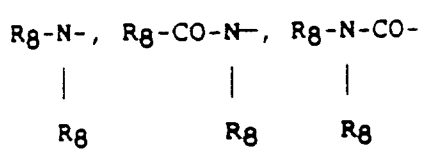

dans lesdites formules, $R_8$ ayant la signification donnée précédemment ; lesdits radicaux pouvant être polycycliques de type "pontés".
3° - un radical aliphatique acyclique, saturé ou insaturé, linéaire ou ramifié, tel que précisé sous 1°, porteur d'un substituant cyclique : ledit radical aliphatique acyclique pouvant être relié au cycle par un lien valentiel ou

par l'un des groupes suivants :
-O-, -CO-, -COO-,

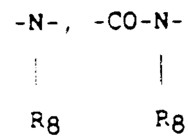

dans lesdites formules, $R_8$ ayant la signification donnée précédemment.

**17.** Procédé selon la revendication 16 caractérisé par le fait que le dérivé halogéné répond à ta formule (III) dans laquelle le radical $R_7$ représente un radical aliphatique acyclique, saturé ou insaturé, linéaire ou ramifié, porteur d'un substituant cyclique choisi parmi :

a - un radical cycloaliphatique saturé ou insaturé, monocyclique ou polycyclique tel que mentionné sous 2° dans la revendication 16,
b - un radical phényle, tolyle, xylyle, éventuellement porteur d'un ou plusieurs substituants Y ou Z.
c - un radical hétérocyclique monocyclique, saturé, insaturé ou aromatique et comportant en tant qu'hétéroatomes un ou plusieurs atomes d'oxygène, d'azote ou de soufre, contenant 4 à 6 atomes dans le cycle : ledit radical pouvant être porteur d'un ou plusieurs substituants Y ou Z.
d - un radical constitué par un enchaînement de 2 à 4 groupes tels que définis aux paragraphes a et/ou b et/ou c, liés entre eux par un lien valentiel et/ou par un des groupes suivants :
-O-, -S-,

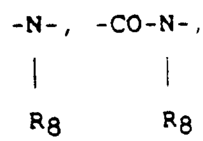

-CO-, -COO-, $-SO_2-$, et/ou encore au moins un groupe de type alcoylène ou alcoylidène ayant de 1 à 4 atomes de carbone ; dans lesdites formules, $R_8$ représente un atome d'hydrogène, un radical alkyle ayant de 1 à 4 atomes de carbone, un radical cyclohexyle ou phényle.
e - un radical aromatique constitué par un ensemble de 2 à 3 cycles aromatiques carbocycliques et/ou hétérocycliques et formant entre eux des systèmes orthocondensés.

**18.** Procédé selon la revendication 15 caractérisé par le fait que le dérivé halogéné répond à la formule (III) dans laquelle $R_7$ représente :

- un radical alkyle linéaire ou ramifié ayant de 1 à 8 atomes de carbone,
- un radical alcényle linéaire ou ramifié ayant de 2 à 8 atomes de carbone et comportant une double liaison éthylénique en β du groupe X,
- un radical cyclohexyle éventuellement substitué par un radical alcoyle linéaire ou ramifié ayant de 1 à 4 atomes de carbone,
- un radical arylalkyle tel que symbolisé par la formule suivante

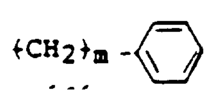

dans laquelle m est un noire entier allant de 1 à 4, de préférence, égal à 1.

**19.** Procédé selon la revendication 15 caractérisé par le fait que le dérivé halogéné est choisi parmi :

- le bromure d'allyle
- le chlorure d'allyle
- le bromure de benzyle

- le chlorure de benzyle
- le bromure d'isopropyle
- le chlorure de crotyle
- le chloro-1 butène-2
- le bromure de cyclohexyle

**20.** Procédé selon la revendication 1 caractérisé par le fait que le catalyseur de transfert de phase est un sel d'onium.

**21.** Procédé selon la revendication 20 caractérisé par la fait que le catalyseur de transfert de phase est un sel d'onium dont l'onium répond à l'une des formules suivantes :

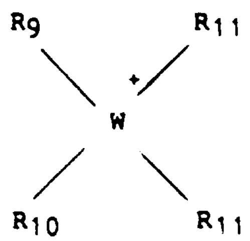

(IV)

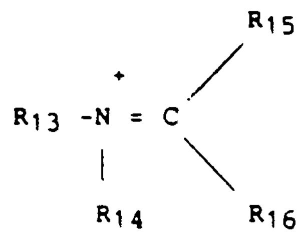

(V)

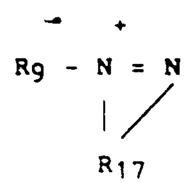

(V bis)

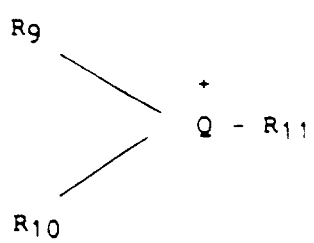

(VI)

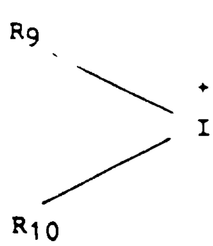

(VII)

dans lesdites formules :

- W représente N, P ou As ;
- Q représente S, O, Se, S=O ou C ;
- $R_9$, $R_{10}$, $R_{11}$ et $R_{12}$, identiques ou différents représentent :

  . un radical alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;
  . un radical alcényle, linéaire ou ramifié, ayant 2 à 12 atomes de carbone ;
  . un radical aryle ayant 6 à 10 atomes de carbone, éventuellement substitué par un ou plusieurs groupements ou atomes alkyle ayant 1 à 4 atomes de carbone, alkoxy, alkoxycarbonyle, le radical alkoxy ayant 1 à 4 atomes de carbone ou halogène,
  . deux desdits radicaux $R_9$ à $R_{12}$ pouvant former ensemble un radical alkylène, alcénylène ou alcadiénylène, linéaire ou ramifié ayant de 3 à 6 atomes de carbone ;

- $R_{13}$, $R_{14}$, $R_{15}$, $R_{16}$ sont identiques ou différents et représentent :

  . un radical alkyle linéaire ou ramifié contenant de 1 à 4 atomes de carbone ;
  . les radicaux-$R_{15}$ et $R_{16}$ pouvant former ensemble un radical alkylène contenant de 3 à 6 atomes de carbone ;
  . les radicaux $R_{14}$ et $R_{15}$ ou $R_{14}$ et $R_{16}$ pouvant former ensemble un radical alkylène, alcénylène ou alcadiènylène, contenant 4 atomes de carbone et constituant avec l'atome d'azote un hétérocycle azoté ;

- $R_{17}$ représente un radical divalent formant avec les 2 atomes d'azote un cycle ayant 4 à 6 atomes pouvant comporter un ou plusieurs atomes d'azote, de soufre et/ou d'oxygène, ledit cycle pouvant être substitué par un ou plusieurs radicaux tels que $R_9$.

**22.** Procédé selon ta revendication 21 caractérisé par le fait que l'anion desdits sels d'onium est choisi parmi les ions : $F^-$, $ClO_4$-, $PF_6^-$, $BF_4^-$, $SnCl_6^-$, $SbCl_6^-$, $B(Ph)_4^-$, $PO_4^{3-}$, $HPO_4^{2-}$, $N_2PO_1^-$, $CH_3SO_3^-$, Ph-$SO_3^-$, $HSO_4^-$, $NO_3$-, $SO_4^{2-}$, $Cl^-$, $Br^-$, $I^-$, $OH^-$, Ph représentant un radical phényle ; l'anion desdits sels d'onium étant choisi de préférence parmi les ions $Br^-$et $Cl^-$.

**23.** Procédé selon l'une des revendications 21 et 22 caractérisé par le fait que l'onium est un ion ammonium quaternaire, un ion phosphonium quaternaire, un ion sulfonium ou un ion iodonium.

**24.** Procédé selon l'une des revendications 21 à 23 caractérisé par le fait que le catalyseur de transfert de phase est choisi parmi : le chlorure ou le bromure de tributylbenzylammonium ou phosphonium, le chlorure ou le bromure de tétraméthylammonium ou phosphonium, le chlorure ou le bromure de tétraéthylammonium ou phosphonium, le chlorure ou le bromure de tétrabutylammonium ou phosphonium.

**25.** Procédé selon l'une des revendications 1 à 24 caractérisé par le fait que le rapport molaire entre le noire de fonctions carboxylate de l'acide carboxylique et le nombre d'atomes d'halogène réagissants du dérivé halogéné varie entre 0,5 et 2,0, et est de préférence, égal à 1,0.

**26.** Procédé selon l'une des revendications 1 à 25 caractérisé par le fait que le rapport molaire entre le catalyseur de transfert de phase et le sel de l'acide carboxylique varie entre 0,01 et 0,50, de préférence, entre 0,05 et 0,2.

**27.** Procédé selon l'une des revendications 1 à 26 caractérisé par le fait que la température réactionnelle varie entre 70°C et 90°C.

## Claims

**1.** A process for esterifying an aromatic carboxylic acid salt, characterized in that it consists of carrying out a reaction, in the absence of any organic solvent, between a salt of an aromatic carboxylic acid and a halogenated derivative of an aliphatic or cycloaliphatic hydrocarbon or an aliphatic hydrocarbon carrying a cyclic substituent, in the presence of a phase transfer catalyst and in the presence of water: the aromatic carboxylic acid salt being gradually introduced into a reaction mixture comprising the halogenated derivative and the catalyst, the reaction taxing place at a temperature of at most 120°C, preferably in the range 50°C to 120°C, leading to a medium comprising an aqueous phase and an organic phase comprising the carboxylic acid ester.

**2.** A process according to claim 1, characterized in that the carboxylic acid salt has the following formula (I):

$$[Q\text{-}COO\text{-}]_w\ M^{w+} \qquad (I)$$

where:

- Q is an aromatic hydrocarbon radical which is optionally substituted, containing 1 to 40 carbon atoms;
- M is a metal cation;
- w is the valency of metal M.

**3.** A process according to claim 1 or claim 2, characterized in that the carboxylic acid salt has formula (I) in which Q is an aromatic hydrocarbon radical which is optionally substituted containing 1 to 20 carbon atoms, and M is a cation of a metal from group Ia or IIa of the periodic table, or an ammonium radical.

**4.** A process according to any one of claims 1 to 3, characterized in that the carboxylic acid salt has formula (I) in which residue Q is a hydrocarbon radical, which may or may not be substituted, which is a carbocyclic, heterocyclic, monocyclic or polycyclic radical.

**5.** A process according to any one of claims 1 to 4, characterized in that the carboxylic acid salt has formula (I) in which Q is an aromatic hydrocarbon residue with general formula (II):

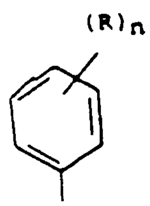

(II)

where:

- n is a while number from 0 to 5, preferably 0 to 3;
- R is $R_1$, one of the following groups or functions:
  - a linear or branched alkyl radical containing 1 to 6 carbon atoms, preferably 1 to 4 carbon atoms;
  - a linear or branched alkenyl radical containing 2 to 6 carbon atoms, preferably 2 to 4 carbon atoms;
  - a linear or branched alkoxy radical containing 1 to 6 carbon atoms, preferably 1 to 4 carbon atoms;
  - a radical with formula

$$R_2\text{-}OH$$

$$R_2\text{-}COOM$$

$$R_2\text{-}CHO$$

$$R_2\text{-}NO_2$$

$$R_2\text{-}CN$$

$$R_2\text{-}NH_2$$

$$R_2\text{-}SH$$

$$R_2\text{-}X$$

$$R_2\text{-}CF_3$$

where $R_2$ is a valence bond or a saturated or unsaturated, linear or branched divalent hydrocarbon radical containing 1 to 4 carbon atoms; X is a halogen atom, preferably a chlorine, bromine or fluorine atom, and M has the meaning hereinbefore defined;

- R is $R_3$, one of the following more complex radicals:
  - a

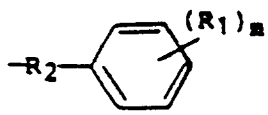

radical where $R_1$ and $R_2$ have the meanings hereinbefore defined and m is a whole number from 0 to 3;
  - a radical $-R_2$-A-$R_4$ where $R_2$ has the meaning hereinbefore defined, $R_4$ is a linear or branched alkyl radical containing 1 to 6 carbon atoms, preferably 1 to 4 carbon atoms, or a radical

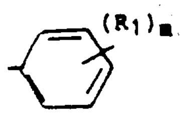

and A is one of the following groups:
—O—, —CO-, —COO

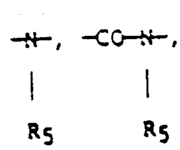

—, —S—, —$SO_2$—, where $R_5$ is a hydrogen atom or a linear or branched alkyl radical containing 1 to 4 carbon atoms, preferably a methyl or ethyl radical.

6. A process according to claim 5, characterized in that the carboxylic acid salt has formula (I) in which Q is an aromatic hydrocarbon residue with general formula (II) where n is greater than 1, radicals R are identical or different and 2 successive atoms of the benzene ring can be connected together by an extranuclear ketal bridge, preferably a methylenedioxy or ethylenedioxy radical.

7. A process according to claim 5, characterized in that the carboxylic acid salt has general formula (I) in which Q is an aromatic hydrocarbon residue with general formula (II) where:

- n equals 0, 1, 2 or 3;
- R is one of the following groups or functions:
  - a linear or branched alkyl radical containing 1 to 4 carbon atoms;
  - a linear or branched alkoxy radical containing 1 to 4 carbon atoms;
  - a methylene- or ethylenedioxy radical;
  - an -OH group;
  - a -CHO group;
  - a phenyl or benzyl radical;
  - a halogen atom.

8. A process according to any one of claims 1 to 4, characterized in that the carboxylic acid salt has general formula (I) where Q is a naphthalene residue; said cycles may be substituted by 1 to 4 radicals $R_1$, preferably 1 to 3, $R_1$ having the meanings hereinbefore defined in claim 5.

9. A process according to any one of claims 1 to 4, characterized in that the carboxylic acid salt has formula (I) where

Q is an aromatic heterocyclic residue in particular containing 5 or 6 atoms in the ring, including 1 or 2 heteroatoms such as nitrogen, sulphur or oxygen atoms or an aromatic polycyclic heterocyclic residue; the carbon atoms of said cycles can optionally be substituted, either completely or in part thereof, only by radicals $R_1$, $R_1$ having the meaning hereinbefore defined in claim 5.

**10.** A process according to any one of claims 1 to 9, characterized in that the carboxylic acid salt with formula (I) is selected from the following carboxylic acids:

- aromatic heterocyclic carboxylic acids;
- aromatic carbocyclic carboxylic acids;
- aromatic hydroxyacids;
- aromatic alkoxy- and phenoxyacids;
- aromatic oxoacids;
- aromatic acyloxyacids;
- aromatic amidoacids.

**11.** A process according to any one of claims 1 to 4, characterized in that the carboxylic acid salt with general formula (I) is selected from aromatic heterocyclic carboxylic acids such as furane carboxylic acids, thiophene carboxylic acids, pyrrole carboxylic acids, pyrazine carboxylic acids, nicotinic acid, isonicotimc acid, picolinic acid, piperonylic acid; aromatic carboxylic acids such as benzoic acid, phthalic acid, isophthalic acid, terephthalic acid, naphthalene carboxylic acids, or toluic acids.

**12.** A process according to any one of claims 1 to 4, characterized in that the carboxylic acid salt with general formula (I) is selected from the following hydroxybenzoic acids: 2-hydroxybenzoic acid (salicylic acid), 3-hydroxybenzoic acid, 4-hydroxybenzoic acid, 3-methylsalicylic acid, 4-methylsalicylic acid, 5-methylsalicylic acid, 3-hydroxy-4-methylbenzoic acid, 3-methoxysalicylic acid, 4-methoxysalicylic acid, 5-methoxysalicylic acid, 3-hydroxy-4-benzoic acid (isovanillic acid), 4-hydroxy-3-methoxybenzoic acid (vanillic acid), 3-hydroxy-4,5-dimethoxybenzoic acid, 4-hydroxy-3,5-dimethoxybenzoic acid (syringic acid), 5-hydroxyisophthalic acid, 3-aminosalicylic acid, 4-aminosalicylic acid, 5-aminosalicylic acid, 3-hydroxy-2-aminobenzoic acid, 3-nitrosalicylic acid, 3-hydroxy-4-nitrobenzoic acid, 4-hydroxy-3-nitrobenzoic acid, 3-hydroxy-4-methyl-2-nitrobenzoic acid, 3,5-diiodosalicylic acid, 2,3-dihydroxybenzoic acid, 2,4-dihydroxyberzoic acid, 2,5-dihydroxybenzoic acid, 2,6-dihydroxybenzoic acid, 3,4-dihydroxybenzoic acid (protocatechuic acid), 3,5-dihydroxybenzoic acid, 3,5-dihydroxy-4-methylbenzoic acid, 2,3,4-trihydroxybenzoic acid, 2,4,6-trthydroxybenzoic acid, and 3,4,5-trihydroxybenzoic acid.

**13.** A process according to any one of claims 1 to 4, characterized in that the carboxylic acid salt with formula (I) is selected from benzoic acid and derivatives such as 4-phenoxybenzoic acid, (4-carboxy-4-phenoxy)benzoic acid, 2-acetylbenzoic acid, 4-acetylbenzoic acid, 2-benzoylbenzoic acid, 4-benzoylbenzoic acid, 2-acetoxybenzoic acid, 4-acetoxybenzoic acid, 2-acetamidobenzoic acid, 3-acetamidobenzoic acid, and N-acetamido-4-benzoic acid.

**14.** A process according to any one of claims 1 to 13, characterized in that the carboxylic acid salt with formula (I) is selected from the following carboxylic acids:

- salicylic acid and 4-hydroxybenzoic acid;
- benzoic acid and its derivatives substituted with a $C_1$-$C_4$ alkyl group, acetoxy, acetamido group;
- nicotinic acid.

**15.** A process according to any one of claims 1 to 14, characterized in that the halogenated derivative has the following formula (III):

$$R_7\text{-}X \qquad \text{(III)}$$

where:

- X is a halogen atom: said halogen atom not being in the vinylic or alkynyl position;
- $R_7$, which may be substituted, is a saturated or unsaturated, linear or branched acyclic aliphatic radical; a saturated or unsaturated, monocyclic or polycyclic cycloaliphatic radical; or a saturated or unsaturated, linear or branched aliphatic radical carrying a cyclic substituent.

16. A process according to claim 15, characterized in that the halogenated derivative has formula (III) in which radical $R_7$ is:

1°- a linear or branched alkyl, alkenyl, alkadienyl, or alkynyl radical preferably containing 1 to 12 carbon atoms; the hydrocarbon chain can optionally be:

- interrupted by one of the following groups:
  -O-, -CO-, -COO-,

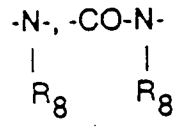

  in which formulae, $R_8$ is a hydrogen atom or an alkyl radical containing 1 to 4 carbon atoms, or a cyclohexyl or phenyl radical;
- and/or carrying one of the following substituents Y:
  an -OH group, a -COOH group, a -CHO group, a-$NO_2$ group a -C≡N group, an amine group which is not quaternisable or N-protected, a halogen atom, preferably chlorine or bromine, or a -$CF_3$ group;

2°- a cycloalkyl or cycloalkenyl radical preferably containing 5 to 7 carbon atoms, optionally carrying one or more substituents Y as hereinbefore defined and/or one or more substituents Z which may be: a linear or branched alkyl radical containing 1 to 4 carbon atoms, a linear or branched alkoxy radical containing 1 to 4 carbon atoms, or a radical with formula:

$R_8$-CO-, $R_8$-COO-, $R_8$-O-CO-,

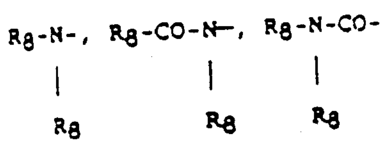

where $R_8$ has the meaning hereinbefore defined: said radicals may be polycyclic "bridged" type radicals;
3°- a saturated or unsaturated, linear or branched acyclic aliphatic radical as defined under 1°, carrying a cyclic substituent: said acyclic aliphatic radical may be connected to the cycle by a valence bond or by one of the following groups:
-O-, -CO-, -COO-,

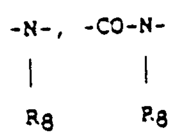

where $R_8$ has the meaning hereinbefore defined.

17. A process according to claim 16, characterized in that the halogenated derivative has formula (III) in which radical $R_7$ is a saturated or unsaturated, linear or branched acyclic aliphatic radical carrying a cyclic substituent selected from:

a. a saturated or unsaturated, monocyclic or polycyclic cycloaliphatic radical as hereinbefore defined under 2° in claim 16;
b. a phenyl, tolyl, or xylyl radical, optionally carrying one or more substituents Y or Z;

c. a saturated, unsaturated or aromatic monocyclic heterocyclic radical containing as heteroatoms one or more oxygen, nitrogen or sulphur atoms, containing 4 to 6 atoms in the cycle: said radical may carry one or more substituents Y or Z;

d. a radical constituted by a sequence of 2 to 4 groups as defined in paragraphs a and/or b and/or c, connected by a valence bond and/or by one of the following groups:

-O-, -S-,

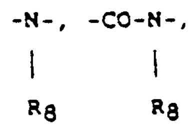

-CO-, -COO-, $-SO_2$—, and/or at least one alkoylene or alkoylidene group containing 1 to 4 carbon atoms; in said formulae, $R_8$ is a hydrogen atom, an alkyl radical containing 1 to 4 carbon atoms, a cyclohexyl or a phenyl radical;

e. an aromatic radical constituted by an assembly of 2 to 3 aromatic carboxylic and/or heterocyclic radicals and forming between them orthocondensed systems.

**18.** A process according to claim 15, characterized in that the halogenated derivative has formula (III) in which $R_7$ is:

- a linear or branched alkyl radical containing 1 to 8 carbon atoms;
- a linear or branched alkenyl radical containing 2 to 8 carbon atoms and containing an ethylenic double bond in the β position from group X;
- a cyclohexyl radical which may be substituted by a linear or branched alkoyl radical containing 1 to 4 carbon atoms;
- an arylalkyl radical as shown in the following formula:

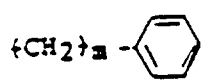

where m is a whole number from 1 to 4, preferably 1.

**19.** A process according to claim 15, characterized in that the halogenated derivative is selected from:

- allyl bromide;
- allyl chloride;
- benzyl bromide;
- benzyl chloride;
- isopropyl bromide;
- crotyl chloride;
- 1-chloro-2-butene;
- cyclohexyl bromide.

**20.** A process according to claim 1, characterized in that the phase transfer catalyst is an onium salt.

**21.** A process according to claim 20, characterized in that the phase transfer catalyst is an onium salt, in which the onium has one of the following formulae:

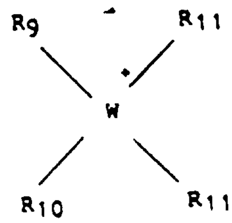

(IV)

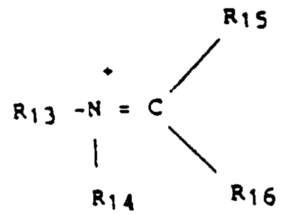

(V)

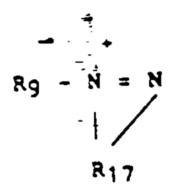

(V bis)

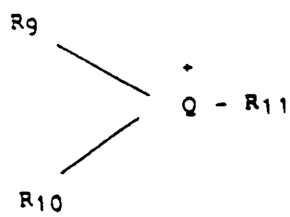

(VI)

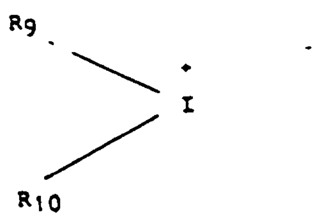

(VII)

where:

- W is N, P or As;
- Q is S, O, Se, S=O or C;
- $R_9$, $R_{10}$, $R_{11}$ and $R_{12}$, which may be identical or different, are:
    - a linear or branched alkyl radical containing 1 to 16 carbon atoms, optionally substituted by one or more phenyl, hydroxyl, halogen, nitro, alkoxy or alkoxycarbonyl groups or atoms, the alkoxy groups containing 1 to 4 carbon atoms;
    - a linear or branched alkenyl radical containing 2 to 12 carbon atoms;
    - an aryl radical containing 6 to 10 carbon atoms, optionally substituted by one or more alkyl groups containing 1 to 4 carbon atoms, alkoxy groups or alkoxycarbonyl groups, the alkoxy radical containing 1 to 4 carbon atoms, or a halogen atom;
    - two of said radicals $R_9$ to $R_{12}$ may together form a linear or branched alkylene, alkenylene or alkadienylene radical containing 3 to 6 carbon atoms;

- $R_{13}$, $R_{14}$, $R_{15}$, $R_{16}$ may be identical or different and are:
  - a linear or branched alkyl radical containing 1 to 4 carbon atoms;
  - radicals $R_{15}$ and $R_{16}$ may together form an alkylene radical containing 3 to 6 carbon atoms;
  - radicals $R_{14}$ and $R_{15}$ or $R_{14}$ and $R_{16}$ may together form an alkylene, alkenylene or alkadienylene radical containing 4 carbon atoms and constituting a nitrogen-containing heterocycle with the nitrogen atom;
- $R_{17}$ is a divalent radical forming with the 2 nitrogen atoms, a cycle containing 4 to 6 atoms which may contain one or more nitrogen, sulphur and/or oxygen atoms, said cycle possibly being substituted by one or more radicals such as $R_9$.

**22.** A process according to claim 21, characterized in that the anion of said onium salts is selected from the following ions: $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, $Ph\text{-}SO_3^-$, $HSO_4^-$, $NO_3^-$, $SO_4^{2-}$, $Cl^-$, $Br^-$, $I^-$, $OH^-$, Ph representing a phenyl radical; the anion of said onium salts preferably being selected from the ions $Br^-$ and $Cl^-$.

**23.** A process according to claim 21 and 22, characterized in that the onium is a quaternary ammonium ion, a quaternary phosphonium ion, a sulphonium ion or an iodonium ion.

**24.** A process according to any one of claims 21 to 23, characterized in that the phase transfer catalyst is selected from : tributylbenzylammonium or phosphonium chloride or bromide, tetramethylammonium or phosphonium chloride or bromide, tetraethylammonium or phosphonium chloride or bromide, and tetrabutylammonium or phosphonium chloride or bromide.

**25.** A process according to any one of claims 1 to 24, characterized in that the molar ratio between the number of carboxylate functions of the carboxylic acid and the number of reacting halogen atoms of the halogenated derivative is from 0.5 to 2.0, and preferably equals 1.0.

**26.** A process according to any one of claims 1 to 25, characterized in that the molar ratio between the phase transfer catalyst and the carboxylic acid salt is from 0.01 to 0.50, preferably 0.05 to 0.2.

**27.** A process according to any one of claims 1 to 26, characterized in that the reaction temperature is from 70°C to 90°C.

## Patentansprüche

**1.** Verfahren zur Veresterung eines Salzes einer aromatischen Carbonsäure, dadurch gekennzeichnet, daß man in Abwesenheit jedes organischen Lösungsmittels und in Gegenwart eines Phasentransferkatalysators sowie in Gegenwart von Wasser eine Reaktion zwischen einem Salz, einer aromatischen Carbonsäure und einem Halogenderivat eines aliphatischen, cycloaliphatischen oder aliphatischen Kohlenwasserstoffs mit einem ringförmigen Substituenten durchführt, wobei das aromatische Carbonsäuresalz nach und nach in die Reaktionsmischung eingetragen wird, welche das Halogenderivat und den Katalysator umfaßt, und die Reaktion bei einer Temperatur von höchstens 120 °C, vorzugsweise zwischen 50 °C und 120 °C, durchgeführt wird, was zu einem Milieu führt, das eine wäßrige Phase und eine organische Phase enthält, die wiederum den Carbonsäureester enthält.

**2.** Verfahren nach Anspruch 1, dadurch gekennzeichnet, daß das Carbonsäuresalz der folgenden Formel (I)

$$[Q\text{ - }COO]_w\, M^{w+} \qquad (I)$$

entspricht, wobei in der Formel (I)

- Q einen gegebenenfalls substituierten aromatischen Kohlenwasserstoffrest mit 1 bis 40 Kohlenstoffatomen entspricht,
- M ein Metallkation darstellt und
- w die Wertigkeit des Metalls M bezeichnet.

**3.** Verfahren nach Anspruch 1 oder 2, dadurch gekennzeichnet, daß das Carbonsäuresalz der Formel (I) entspricht, in der Q einen gegebenenfalls substituierten aromatischen Konlenwasserstoffrest mit 1 bis 20 Kohlenstoffatomen

darstellt und M ein Metallkation der Gruppe Ia oder IIa des Periodensystems oder einen Ammoniumrest darstellt.

**4.** Verfahren nach einem der Ansprüche 1 bis 3, dadurch gekennzeichnet, daß das Carbonsäuresalz der Formel (I) entspricht, in der der Rest Q einen gegebenenfalls substituierten Kohlenwasserstoffrest darstellt, der ein monocyclischer oder polycyclischer, carbocyclischer oder heterocyclischer Rest ist.

**5.** Verfahren nach einem der Ansprüche 1 bis 4, dadurch gekennzeichnet, daß das Carbonsäuresalz der Formel (I) entspricht, in der Q einen aromatischen Kohlenwasserstoffrest darstellt, der der allgemeinen Formel (II)

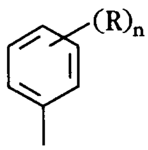

(II)

entspricht, wobei in der Formel (II)

— n eine ganze Zahl von 0 bis 5, vorzugsweise von 0 bis 3, ist;
— R einen Rest $R_1$ darstellt, der eine der folgenden Gruppen oder Funktionen bezeichnet:

- einen linearen oder verzweigten Alkylrest mit 1 bis 6 Kohlenstoffatomen, vorzugsweise mit 1 bis 4 Kohlenstoffatomen,
- einen linearen oder verzweigten Alkenylrest mit 2 bis 6 Kohlenstoffatomen, vorzugsweise mit 2 bis 4 Kohlenstoffatomen,
- einen linearen oder verzweigten Alkoxyrest mit 1 bis 6 Kohlenstoffatomen, vorzugsweise mit 1 bis 4 Kohlenstoffatomen,
- einen Rest der Formel

$-R_2-OH$

$-R_2-COOM$

$-R_2-CHO$

$-R_2-NO_2$

$-R_2-CN$

$-R_2-NH_2$

$-R_2-SH$

$-R_2-X$

$-R_2-CF_3$

wobei in diesen Formeln $R_2$ eine Valenzbindung oder einen gesättigten oder ungesättigten, linearen oder verzweigten, zweiwertigen Kohlenwasserstoffrest mit 1 bis 4 Kohlenstoffatomen darstellt; X ein Halogenatom, vorzugsweise ein Chlor-, Brom- oder Fluoratom darstellt; und M die zuvor angegebene Bedeutung besitzt;

— R einen Rest $R_3$ darstellt, der einen der folgenden komplexen Reste bezeichnet:

- einen Rest

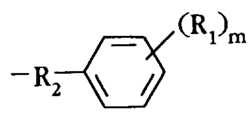

in dem $R_1$ und $R_2$ die zuvor angegebene Bedeutung haben und m eine ganze Zahl von 0 bis 3 darstellt,

- einen Rest $-R_2-A-R_4$, bei dem $R_2$ die zuvor angegebene Bedeutung hat, wobei $R_4$ einen linearen oder verzweigten Alkylrest mit 1 bis 6 Kohlenstoffatomen, vorzugsweise mit 1 bis 4 Kohlenstoffatomen, oder einen Rest

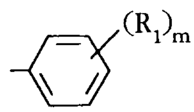

darstellt und A eine der folgenden Gruppen symbolisiert
-O-, -CO-, -COO-,

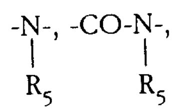

-S-, $-SO_2-$, wobei in diesen Formeln $R_5$ ein Wasserstoffatom oder einen linearen oder verzweigten Alkylrest mit 1 bis 4 Kohlenstoffatomen, vorzugsweise einen Methyl- oder Ethylrest, darstellt.

**6.** Verfahren nach Anspruch 5, dadurch gekennzeichnet, daß das Carbonsäuresalz der Formel (I) entspricht, in der Q einen aromatischen Kohlenwasserstoffrest darstellt, der der allgemeinen Formel (II) entspricht, in der n größer als 1 ist, die Reste R identisch oder verschiedensind und zwei aufeinanderfolgende Kohlenstoffatome im Benzolring untereinander über eine Ketalbrücke verbunden sein können, vorzugsweise über einen außerhalb des Rings befindlichen Methylendioxy- oder Ethylendioxyrest.

**7.** Verfahren nach Anspruch 5, dadurch gekennzeichnet, daß das Carbonsäuresalz der allgemeinen Formel (I) entspricht, in der Q einen aromatischen Kohlenwasserstoffrest darstellt, der der allgemeinen Formel (II) entspricht, in der:

— n 0, 1, 2 oder 3 ist;
— R eine der folgenden Gruppen oder Funktionen darstellt:

- einen linearen oder verzweigten Alkylrest mit 1 bis 4 Kohlenstoffatomen,
- einen linearen oder verzweigten Alkoxyrest mit 1 bis 4 Kohlenstoffatomen,
- einen Methylendioxy- oder Ethylendioxyrest,
- eine Gruppe -OH,
- eine Gruppe -CHO,
- einen Phenyl- oder Benzylrest,
- ein Halogenatom.

**8.** Verfahren nach einem der Ansprüche 1 bis 4, dadurch gekennzeichnet, daß das Carbonsäuresalz der allgemeinen Formel (I) entspricht, in der Q einen Naphthalinrest darstellt, wobei die Ringe mit 1 bis 4 Resten $R_1$, vorzugsweise mit 1 bis 3 Resten $R_1$, substituiert sein können, wobei $R_1$ die zuvor in Anspruch 5 genannten Bedeutungen hat.

**9.** Verfahren nach einem der Ansprüche 1 bis 4, dadurch gekennzeichnet, daß das Carbonsäuresalz der Formel (I) entspricht, in der Q einen aromatischen heterocyclischen Rest mit insbesondere 5 oder 6 Atomen im Ring, hierunter 1 oder 2 Heteroatome wie Stickstoff, Schwefel und Sauerstoff, oder einen aromatischen, polycyclischen heterocyclischen Rest darstellt, wobei die Kohlenstoffatome der Ringe in ihrer Gesamtheit oder nur teilweise gegebenenfalls mit dem Rest $R_1$ substituiert sein können, wobei $R_1$ die zuvor in Anspruch 5 angegebene Bedeutung hat.

**10.** Verfahren nach einem der Ansprüche 1 bis 9, dadurch gekennzeichnet, daß das der Formel (I) entsprechende Carbonsäuresalz ausgewählt ist aus den folgenden Carbonsäuren:

— aromatischen heterocyclischen Carbonsäuren,
— aromatischen carbocyclischen Carbonsäuren,
— aromatischen Hydroxysäuren,
— aromatischen Alkoxy- und Phenoxysäuren,
— aromatischen Oxosäuren,
— aromatischen Acyloxysäuren,
— aromatischen Amidosäuren.

**11.** Verfahren nach einem der Ansprüche 1 bis 4, dadurch gekennzeichnet, daß das der allgemeinen Formel (I) entsprechende Carbonsäuresalz ausgewählt ist aus aromatischen heterocyclischen Carbonsäuren wie den Furancarbonsäuren, den Thiophencarbonsäuren, den Pyrrolcarbonsäuren, den Pyrazincarbonsäuren, der Nikotinsäure, der Isonikotinsäure, der Picolinsäure, der Piperonylsäure; aus aromatischen carbocyclischen Carbonsäuren wie der Benzoesäure, der Phthalsäure, der Isophthalsäure, der Terephthalsäure, den Naphthalincarbonsäuren und den Toluolsäuren.

**12.** Verfahren nach einem der Ansprüche 1 bis 4, dadurch gekennzeichnet, daß das Carbonsäuresalz, das der allgemeinen Formel (I) entspricht, ausgewählt ist aus den folgenden Hydroxybenzoesäuren: 2-Hydroxybenzoesäure (Salicylsäure), 3-Hydroxybenzoesäure, 4-Hydroxybenzoesäure, 3-Methylsalicylsäure, 4-Methylsalicylsäure, 5-Methylsalicylsäure, 3-Hydroxy-4-methylbenzoesäure, 3-Methoxysalicylsäure, 4-Methoxysalicylsäure, 5-Methoxysalicylsäure, 3-Hydroxy-4-methoxybenzoesäure (Isovanillinsäure), 4-Hydroxy-3-methoxybenzoesäure (Vanillinsäure), 3-Hydroxy-4,5-dimethoxybenzoesäure, 4-Hydroxy-3,5-dimethoxybenzoesäure (Syringinsäure), 5-Hydroxyisophthalsäure, 3-Aminosalicylsäure, 4-Aminosalicylsäure, 5-Aminosalicylsäure, 3-Hydroxy-2-aminobenzoesäure, 3-Nitrosalicylsäure, 3-Hydroxy-4-nitrobenzoesäure, 4-Hydroxy-3-nitrobenzoesäure, 3-Hydroxy-4-methyl-2-nitrobenzoesäure, 3,5-Diiodsalicylsäure, 2,3-Dihydroxybenzoesäure, 2,4-Dihydroxybenzoesäure, 2,5-Dihydroxybenzoesäure, 2,6-Dihydroxybenzoesäure, 3,4-Dihydroxybenzoesäure (Protocatechinsäure), 3,5-Dihydroxybenzoesäure, 3,5-Dihydroxy-4-methylbenzoesäure, 2,3,4-Trihydroxybenzoesäure, 2,4,6-Trihydroxybenzoesäure und 3,4,5-Trihydroxybenzoesäure.

**13.** Verfahren nach einem der Ansprüche 1 bis 4, dadurch gekennzeichnet, daß das Carbonsäuresalz, das der Formel (I) entspricht, ausgewählt ist aus Benzoesäuren und ihren Derivaten, wie z.B. 4-Phenoxybenzoesäure, (4-Carboxy-4-phenoxy)-benzoesäure, 2-Acetylbenzoesäure, 4-Acetylbenzoesäure, 2-Benzoylbenzoesäure, 4-Benzoylbenzoesäure, 2-Acetoxybenzoesäure, 4-Acetoxybenzoesäure, 2-Acetamidobenzoesäure, 3-Acetamidobenzoesäure und N-4-Acetamidobenzoesäure.

**14.** Verfahren nach einem der Ansprüche 1 bis 13, dadurch gekennzeichnet, daß das Carbonsäuresalz, das der Formel (I) entspricht, ausgewählt ist aus den folgenden Carbonsäuren:

— Salicylsäure und 4-Hydroxybenzoesäure;
— Benzoesäure und ihren Derivaten, die mit einer $C_1$-$C_4$-Alkylgruppe, einer Acetoxygruppe oder einer Acetamidogruppe substituiert sind
— Nikotinsäure.

**15.** Verfahren nach einem der Ansprüche 1 bis 14, dadurch gekennzeichnet, daß das Halogenderivat der folgenden Formel (III) entspricht

$$R_7\text{-}X \qquad \text{(III)}$$

wobei in der Formel (III)

— X ein Halogenatom darstellt, wobei das Halogenatom sich nicht in der Vinyl- oder Alkinylposition befinden darf;

— $R_7$, gegebenenfalls substituiert, einen linearen oder verzweigten, gesättigten oder ungesättigten, acyclischen aliphatischen Rest; einen monocyclischen oder polycyclischen, gesättigten oder ungesättigten, cycloaliphatischen Rest; einen linearen oder verzweigten, gesättigten oder ungesättigten aliphatischen Rest mit einem ringförmigen Substituenten darstellt.

**16.** Verfahren nach Anspruch 15, dadurch gekennzeichnet, daß das Halogenderivat der Formel (III) entspricht, in der der Rest $R_7$ darstellt:

1.) - einen linearen oder verzweigten Alkyl-, Alkenyl-, Alkadienyl- oder Alkinylrest mit vorzugsweise 1 bis 12 Kohlenstoffatomen, wobei die Kohlenwasserstoffkette gegebenenfalls

- unterbrochen sein kann von einer der folgenden Gruppen:
  -O-, -CO-, -COO-,

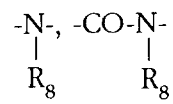

  wobei in diesen Formeln $R_8$ ein Wasserstoffatom oder einen Alkylrest mit 1 bis 4 Kohlenstoffatomen, einen Cyclohexyl- oder einen Phenylrest darstellen,
- und/oder einen der folgenden Substituenten Y enthalten kann: eine OH-Gruppe, eine COOH-Gruppe, eine CHO-Gruppe, eine $NO_2$-Gruppe, eine C ≡ N-Gruppe, eine nicht quaternisierbare oder N-geschützte Amingruppe, ein Halogenatom, vorzugsweise Chlor oder Brom, oder eine $CF_3$-Gruppe.

2.) - einen Cycloalkyl- oder Cycloalkenylrest mit vorzugsweise 5 bis 7 Kohlenstoffatomen, der gegebenenfalls ein oder mehrere Substituenten Y mit der zuvor angegebenen Bedeutung und/oder einen oder mehrere Substituenten Z enthält, wobei Z sein kann: ein linearer verzweigter Alkylrest mit 1 bis 4 Kohlenstoffatomen, ein linearer oder verzweigter Alkoxyrest mit 1 bis 4 Kohlenstoffatomen, ein Rest der Formel

$R_8$-CO-, $R_8$-COO, $R_8$-O-CO-

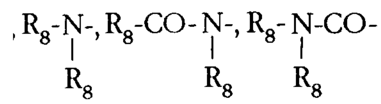

wobei in diesen Formeln $R_8$ die zuvor angegebene Bedeutung hat und die Reste polycyclisch vom „verbrückten" Typ sein können;

3.) - einen linearen oder verzweigten, gesättigten oder ungesättigten, acyclischen aliphatischen Rest, wie zuvor unter Aufzählungspunkt 1.) genannt, der einen ringförmigen Substituenten enthält, wobei der acyclische aliphatische Rest mit dem Ring über eine Valenzbindung oder eine der folgenden Gruppen verbunden sein kann

-O-, -CO-, -COO-,

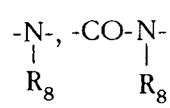

wobei in diesen Formeln $R_8$ die zuvor angegebene Bedeutung hat.

**17.** Verfahren nach Anspruch 16, dadurch gekennzeichnet, daß das Halogenderivat der Formel (III) entspricht, in der der Rest $R_7$ einen gesättigten oder ungesättigten, linearen oder verzweigten, acyclischen aliphatischen Rest darstellt, der einen ringförmigen Substituenten enthält, der ausgewählt ist aus:

(a) einem monocyclischen oder polycyclischen, gesättigten oder ungesättigten cycloaliphatischen Rest, wie z.B. den unter 2.) in Anspruch 16 genannten;
(b) einem Xylyl-, Tolyl- oder Phenylrest, der gegebenenfalls ein oder mehrere Substituenten Y oder Z enthält;
(c) einem aromatischen, gesättigten oder ungesättigten monocyclischen heterocyclischen Rest, der als Heteroatome ein oder mehrere Sauerstoff-, Stickstoff- oder Schwefelatome enthält und dessen Ring 4 bis 6 Atome umfaßt, wobei der Rest ein oder mehrere Substituenten Y oder Z enthalten kann;
(d) einem Rest, der aus einer Verkettung von 2 bis 4 Gruppen, wie sie zuvor unter den Aufzählungspunkten (a) und/oder (b) und/oder (c) definiert worden sind, besteht, die untereinander über eine Valenzbindung verbunden sind und/oder über die folgenden Gruppen

-O-, -S-,

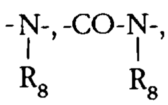

-CO-, -COO-, $SO_2$-, und/oder aber auch mindestens eine Gruppe vom Alkyltyp oder Alkylidentyp mit 1 bis 4 Kohlenstoffatomen, wobei in den Formeln der Rest $R_8$ ein Wasserstoffatom, einen Alkylrest mit 1 bis 4 Kohlenstoffatomen oder einen Cyclohexyl- oder Phenylrest darstellt;
(e) einem aromatischen Rest, der aus einer Gesamtheit von 2 bis 3 carbocyclischen und/oder heterocyclischen aromatischen Ringen besteht, die untereinander orthokondensierte Systeme bilden.

**18.** Verfahren nach Anspruch 15, dadurch gekennzeichnet, daß das Halogenderivat der Formel (III) entspricht, in der der Rest $R_7$ darstellt:

- einen linearen oder verzweigten Alkylrest mit 1 bis 8 Kohlenstoffatomen,
- einen linearen oder verzweigten Alkenylrest mit 2 bis 8 Kohlenstoffatomen und mit einer ethylenischen Doppelbindung in β-Position zur Gruppe X,
- einen gegebenenfalls mit einem linearen oder verzweigten Alkylrest mit 1 bis 4 Kohlenstoffatomen substituierten Cyclohexylrest,
- einen Arylalkylrest, der durch die folgende Formel

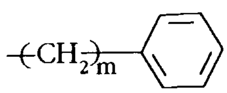

dargestellt wird, in der m eine ganze Zahl von 1 bis 4, vorzugsweise 1, ist.

**19.** Verfahren nach Anspruch 15, dadurch gekennzeichnet, daß das Halogenderivat ausgewählt ist aus:

- Allylbromid,
- Allylchlorid,
- Benzylbromid,
- Benzylchlorid,
- Isopropylbromid,
- Crotylchlorid,
- 1-Chlor-2-buten,
- Cyclohexylbromid.

**20.** Verfahren nach Anspruch 1, dadurch gekennzeichnet, daß der Phasentransferkatalysator ein Oniumsalz ist.

**21.** Verfahren nach Anspruch 20, dadurch gekennzeichnet, daß der Phasentransferkatalysator ein Oniumsalz ist,

wobei Onium einer der folgenden Formeln entspricht:

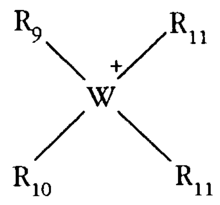

(IV)

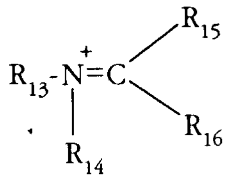

(V)

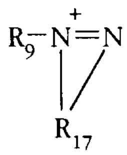

(V bis)

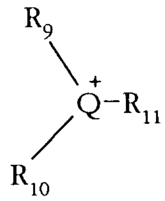

(VI)

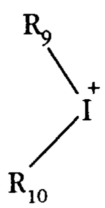

(VII)

wobei in diesen Formeln:

- W N, P oder As darstellt;
- Q S, O, Se, S=O oder C darstellt;
- $R_9$, $R_{10}$, $R_{11}$ und $R_{12}$, identisch oder verschieden, darstellen:
  - einen linearen oder verzweigten Alkylrest mit 1 bis 16 Kohlenstoffatomen, der gegebenenfalls substituiert ist mit einem oder mehreren Gruppen oder Atomen wie Phenyl, Hydroxyl, Halogen, Nitro, Alkoxy oder Alkoxycarbonyl, wobei die Alkoxygruppen 1 bis 4 Kohlenstoffatome aufweisen;
  - einen linearen oder verzweigten Alkenylrest mit 2 bis 12 Kohlenstoffatomen;
  - einen Arylrest mit 6 bis 10 Kohlenstoffatomen, der gegebenenfalls mit einem oder mehreren Gruppen oder Atomen substituiert ist, wie z.B. Alkyl mit 1 bis 4 Kohlenstoffatomen, Alkoxy, Alkoxycarbonyl, einem Alkoxyrest mit 1 bis 4 Kohlenstoffatomen oder Halogen;
  - wobei zwei der Reste $R_9$ bis $R_{12}$ zusammen einen linearen oder verzweigten Alkylen-, Alkenylen- oder Alkadienylenrest mit 3 bis 6 Kohlenstoffatomen bilden können;

— $R_{13}$, $R_{14}$, $R_{15}$, $R_{16}$, identisch oder verschieden, darstellen:

- einen linearen oder verzweigten Alkylrest mit 1 bis 4 Kohlenstoffatomen;
- die Reste $R_{15}$ und $R_{16}$ zusammen einen Alkylenrest mit 3 bis 6 Kohlenstoffatomen bilden können;
- die Reste $R_{14}$ und $R_{15}$ oder $R_{14}$ und $R_{16}$ zusammen einen Alkylen-, Alkenylen- oder Alkadienylenrest mit 4 Kohlenstoffatomen darstellen, der mit einem Stickstoffatom einen stickstoffhaltigen Heterozyklus bildet;

— $R_{17}$ einen zweiwertigen Rest darstellt, der mit 2 Stickstoffatomen einen Ring mit 4 bis 6 Atomen bildet, der ein oder mehrere Stickstoff-, Schwefel- und/oder Sauerstoffatome enthält, wobei der Ring mit einem oder mehreren Resten, wie z.B. $R_9$ substituiert sein kann.

**22.** Verfahren nach Anspruch 21, dadurch gekennzeichnet, daß das Anion der Oniumsalze ausgewählt ist aus den folgenden Ionen: $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, Ph-$SO_3^-$, $HSO_4^-$, $NO_3^-$, $SO_4^{2-}$, $Cl^-$, $Br^-$, $I^-$ und $OH^-$, wobei Ph einen Phenylrest darstellt und die Anionen der Oniumsalze vorzugsweise ausgewählt sind aus den Ionen $Br^-$ und $Cl^-$.

**23.** Verfahren nach Anspruch 21 oder 22, dadurch gekennzeichnet, daß das Onium ein quaternäres Ammoniumion, ein quaternäres Phosphoniumion, ein Sulfoniumion oder ein Iodoniumion ist.

**24.** Verfahren nach einem der Ansprüche 21 bis 23, dadurch gekennzeichnet, daß der Phasentransferkatalysator ausgewählt ist aus Tributylbenzylammonium- oder -phosphoniumchlorid oder -bromid, Tetramethylammonium- oder -phosphoniumchlorid oder -bromid, Tetraethylammonium- oder -phosphoniumchlorid oder -bromid und Tetrabutylammonium- oder -phosphoniumchlorid oder -bromid.

**25.** Verfahren nach einem der Ansprüche 1 bis 24, dadurch gekennzeichnet, daß das Molverhältnis zwischen der Anzahl der Carboxylatfunktionen der Carbonsäure und der Anzahl der reagierenden Halogenatome des Halogenderivats zwischen 0,5 und 2,0 variiert und vorzugsweise bei 1,0 liegt.

**26.** Verfahren nach einem der Ansprüche 1 bis 25, dadurch gekennzeichnet, daß das Molverhältnis zwischen Phasentransferkatalysator und Carbonsäuresalz zwischen 0,01 und 0,50 variiert, vorzugsweise zwischen 0,05 und 0,2.

**27.** Verfahren nach einem der Ansprüche 1 bis 26, dadurch gekennzeichnet, daß die Reaktionstemperatur zwischen 70 °C und 90 °C variiert.